(19) Europäisches Patentamt
European Patent Office
Office européen des brevets

(11) EP 1 402 876 B1

(12) # FASCICULE DE BREVET EUROPEEN

(45) Date de publication et mention de la délivrance du brevet:
**27.05.2009 Bulletin 2009/22**

(21) Numéro de dépôt: **03103447.3**

(22) Date de dépôt: **18.09.2003**

(51) Int Cl.:
*A61K 8/87* (2006.01) *A61K 8/85* (2006.01)
*A61K 8/81* (2006.01) *A61K 8/88* (2006.01)
*A61Q 1/10* (2006.01)

(54) **Composition cosmétique comprenant des fibres**

Fasernhaltige kosmetische Zusammensetzung

Cosmetic composition containing fibers

(84) Etats contractants désignés:
**DE ES FR GB IT**

(30) Priorité: **20.09.2002 FR 0211673**

(43) Date de publication de la demande:
**31.03.2004 Bulletin 2004/14**

(73) Titulaire: **L'ORÉAL**
**75008 Paris (FR)**

(72) Inventeur: **JAGER-LEZER, Nathalie**
**91370, VERRIERES-LE-BUISSON (FR)**

(74) Mandataire: **Poulin, Gérard et al**
**c/o Brevalex,**
**3, rue du docteur Lancereaux**
**75008 Paris (FR)**

(56) Documents cités:
**EP-A- 0 445 342**
**EP-A- 1 053 742**
**EP-A- 1 201 221**
**EP-A- 1 208 836**
**US-A- 3 802 841**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 402 876 B1

Printed by Jouve, 75001 PARIS (FR)

## Description

La présente invention concerne une composition cosmétique de maquillage ou de soin des matières kératiniques comprenant des fibres.

L'invention a trait également à l'utilisation de cette composition pour le maquillage des matières kératiniques, notamment des cils, sourcils et cheveux, ainsi qu'à un procédé de maquillage ou de soin cosmétique de ces dernières.

La composition et le procédé de maquillage, selon l'invention, sont plus particulièrement destinés aux fibres kératiniques, notamment sensiblement longitudinales, d'êtres humains, telles que les cils, les sourcils et les cheveux, y compris les faux cils, de préférence la composition et le procédé de maquillage, selon l'invention sont destinés aux cils.

La composition peut être une composition de maquillage, une base de maquillage, une composition à appliquer sur un maquillage, dite encore « top-coat », ou bien encore une composition de traitement cosmétique, de soin, des fibres kératiniques. Plus particulièrement, l'invention porte sur un mascara.

Il est connu, dans ce domaine de la technique, d'employer des fibres dans les compositions de maquillage ou de soin des matières kératiniques pour améliorer leurs propriétés cosmétiques.

Ainsi, il est connu du document JP-A-3 153 613 d'utiliser les fibres dans des compositions de mascara pour conférer un effet d'allongement et d'épaississement aux cils. Les documents JP-A-57 158 714 et JP-A-9 263 518 décrivent des compositions de mascara comprenant des fibres et des polymères de type acrylique en dispersion aqueuse.

De même, les documents JP-A-6 9340 et JP-A-7 179 323 décrivent des compositions de mascara comprenant des fibres et des polymères en dispersion aqueuse.

Le document FR-A-2 817 477 décrit des compositions ou formulations cosmétiques comprenant des fibres à base d'un polymère synthétique ou artificiel, tel que le polypropylène, le PET, le polyamide 6 et le polyamide 66.

Les documents EP-A-1 201 221, EP-A-1 053 742 et EP-A-1 208 836 décrivent des compositions de mascara comprenant des fibres de polyamide, polyoléfine, polyuréthane, polyester ou polyacrylate.

Toutefois, les compositions cosmétiques contenant des fibres de l'art antérieur présentent un certain nombre d'inconvénients, tels qu'un maquillage hétérogène et peu précis.

En particulier, les compositions de mascara contenant des fibres de l'art antérieur ne permettent pas d'obtenir un effet d'allongement optimal des cils après l'application de la composition. En effet, les fibres sont orientées et réparties de manière aléatoire sur les cils et ne se situent pas dans le prolongement des cils.

L'effet obtenu par les compositions de mascara contenant des fibres de l'art antérieur est souvent esthétiquement inacceptable, notamment dans le cas de cils fournis et/ou longs et/ou recourbés, pour lesquels on obtient un aspect des cils, dit « sapin de noël », particulièrement disgracieux.

Il existe donc un besoin pour une composition cosmétique, en particulier une composition de mascara qui permette d'obtenir d'excellentes propriétés cosmétiques, un maquillage homogène et précis.

Il existe notamment un besoin pour une composition de mascara qui procure un allongement parfait, dans une continuité parfaite du cil, un positionnement régulier, non-aléatoire, des fibres exactement dans le prolongement du cil et donc un effet esthétique et d'allongement optimal.

En outre, cette composition doit, de préférence, conserver l'effet d'allongement dans le temps.

Le but de la présente invention est, entre autres, de répondre aux besoins et de satisfaire aux exigences mentionnés plus haut.

Ce but et d'autres encore sont atteints, conformément à l'invention par une composition cosmétique comprenant des fibres rigides, sensiblement rectilignes, d'un polymère synthétique dans un milieu physiologiquement acceptable, dans laquelle le polymère est choisi parmi les polyuréthanes, les polyesters, les polymères acryliques, les polyoléfines, les polyamides non aromatiques et les polyimides-amides aromatiques. Avantageusement, au moins 50 % en nombre, de préférence au moins 75% en nombre, et mieux au moins 90% en nombre des fibres sont telles que l'angle formé entre la tangente à l'axe central longitudinal de la fibre à l'une des extrémités de la fibre et la droite reliant ladite extrémité au point sur l'axe central longitudinal de la fibre correspondant à la moitié de la longueur de fibre soit inférieur ou égal à 15°, et l'angle formé entre la tangente à l'axe central longitudinal de la fibre en un point situé à mi-longueur de la fibre et la droite reliant une des extrémités au point sur l'axe central longitudinal de la fibre correspondant à la moitié de la longueur de fibre soit inférieur ou égal à 15°, pour une même longueur de fibre allant de 0,8 mm à 5 mm, de préférence allant de 1 à 4 mm, de préférence encore de 1 à 3 mm, et mieux de 2 mm.

L'angle, mentionné ci-dessus, est mesuré aux deux extrémités de la fibre et en un point situé à mi-longueur de la fibre, en d'autres termes, on effectue dans ce cas trois mesures et la moyenne des angles mesurés est inférieure ou égale à 15°.

La tangente, en tout point de la fibre, forme un angle inférieur ou égal à 15°.

Dans la présente demande, l'angle formé par la tangente en un point de la fibre est l'angle formé entre la tangente à l'axe central longitudinal de la fibre audit point de la fibre et la droite reliant l'extrémité de la fibre la plus proche dudit point au point sur l'axe central longitudinal de la fibre correspondant à la moitié de la longueur de la fibre.

Généralement les fibres incluses dans la composition ont la même longueur de fibre ou une longueur sensiblement identique.

La composition, selon l'invention, se différencie fondamentalement des compositions de l'art antérieur ; tout d'abord, parmi l'infinité de types de fibres pouvant être incorporés dans une composition cosmétique, telles que les fibres minérales, synthétiques, naturelles, ont été sélectionnées seulement cinq familles spécifiques de fibres qui sont, selon l'invention, des fibres de polymère synthétique, ledit polymère synthétique étant choisi parmi les polyuréthanes, les polyesters, les polymères acryliques, les polyoléfines, les polyamides non aromatiques, et les polyimides-amides (ou polyamides-imides) aromatiques.

Un nombre très réduit de types de polymères synthétiques différents au niveau de leur structure ont donc été choisis pour être incorporés dans les compositions de l'invention.

En outre, les fibres des compositions selon l'invention sont définies par un paramètre particulier se trouvant dans une plage particulière. Ce paramètre est, pour une même longueur de fibre allant de 0,8 mm à 5 mm, de préférence allant de 1 à 4 mm, de préférence encore de 1 à 3 mm, et mieux de 2 mm, l'angle formé par (entre) la tangente à l'axe central, longitudinal de la fibre à l'une de ses extrémités et la droite reliant ladite extrémité au point sur l'axe central longitudinal de la fibre correspondant ou situé à la moitié de la longueur de la fibre qui doit, selon l'invention, être inférieur ou égal à 15°, de préférence inférieur ou égal à 10°, de préférence encore inférieur ou égal à 5°, et l'angle formé entre la tangente à l'axe central longitudinal de la fibre en un point situé à mi-longueur de la fibre et la droite reliant une des extrémités au point sur l'axe central longitudinal de la fibre correspondant à la moitié de la longueur de fibre qui doit, selon l'invention, être inférieur ou égal à 15°, de préférence inférieur ou égal à 10°, de préférence encore inférieur ou égal à 5°. Cette condition d'angle doit, de plus, être satisfaite par au moins un nombre majoritaire des fibres, c'est-à-dire 50% en nombre de fibres, de préférence au moins 75% en nombre des fibres et mieux au moins 90 % en nombre des fibres.

Plus précisément, selon l'invention, lorsque l'on observe au microscope, avec un objectif permettant un grossissement de 2,5 et avec une vision plein champ, un milieu dans lequel sont dispersées les fibres à une concentration des fibres de 1 % en poids, un nombre majoritaire des fibres, c'est-à-dire au moins 50% en nombre des fibres, de préférence au moins 75% en nombre des fibres, et mieux au moins 90 % en nombre des fibres doivent satisfaire à la condition angulaire définie plus haut. La mesure conduisant à la valeur de l'angle est effectuée pour une même longueur de fibres, cette longueur est comprise dans la gamme allant de 0,8 mm à 5 mm, de préférence de 1 à 4 mm, de préférence encore de 1 à 3 mm et mieux de 2 mm. La vision plein champ permet de voir les fibres dans leur intégralité.

Le milieu dans lequel est réalisée l'observation est un milieu dispersant assurant une bonne dispersion des fibres, par exemple de l'eau, un gel aqueux d'argile ou de polyuréthane associatif. On peut même réaliser une observation directe de la composition contenant les fibres.

En fait, les fibres incluses dans les compositions de l'invention peuvent aussi être définies comme étant des fibres rigides, par opposition aux fibres des compositions de l'art antérieur, qui ne sont pas des fibres rigides et forment, de ce fait, des boucles de courbures assez importantes à l'observation au microscope.

En d'autres termes, les fibres des compositions de l'invention, initialement sensiblement droites, lorsqu'elles sont placées dans un milieu dispersant, ne voient pas leur forme sensiblement modifiée, ce qui se traduit par la condition angulaire définie ci-dessus, reflétant une forme que l'on peut qualifier de toujours, sensiblement droite, linéaire, rectiligne, une forme de "bâton".

Cette condition d'angle reflète la rigidité conservée des fibres qui peut difficilement être exprimée par un autre paramètre que celui choisi selon l'invention pour des objets ayant une taille aussi faible que les fibres mise en oeuvre dans les compositions de l'invention.

Plus exactement, la condition d'angle à laquelle doivent satisfaire les fibres des compositions de l'invention illustre la conservation de la forme des fibres, qui demeure sensiblement rectiligne, en raison de la rigidité de la fibre.

Avec les fibres de l'art antérieur, qui ne se présentent pas comme des « bâtonnets droits » et qui ne satisfont donc pas à la condition d'angle des fibres des compositions de l'invention, l'effet d'allongement ne peut être obtenu et n'est donc pas optimisé.

Les fibres de l'art antérieur qui sont souples n'ont pas de forme initialement rectiligne et lorsqu'elles sont placées dans un milieu dispersant tel qu'une composition cosmétique, ces fibres se déforment en formant des boucles, et ne procurent aucun effet d'allongement, et donnent un aspect inesthétique du cil. Pour employer une comparaison triviale, les fibres selon l'invention peuvent être comparées à des spaghettis avant cuisson qui sont rigides, rectilignes et conservent cette forme alors que les fibres de l'art antérieur, souples, pourraient être comparées à des spaghettis cuits qui se déforment, se recourbent, et ne peuvent pas maintenir une forme rectiligne.

Les compositions de l'invention, incorporant les fibres spécifiques décrites plus haut, possèdent d'excellentes propriétés cosmétiques, en particulier elles permettent un maquillage homogène et précis, dans le cas des mascaras. Les fibres des compositions de l'invention ne sont pas disposées de manière aléatoire, mais se placent dans le prolongement des cils. En fait, la composition selon l'invention confère un très bon effet d'allongement aux cils, grâce aux fibres particulières qu'elle contient, et qui se placent parfaitement, exactement, dans le prolongement du cil. La composition de l'invention permet, en fait, de réaliser une véritable « prothèse » du cil, ce qui est totalement impossible avec

les compositions de l'art antérieur, renfermant des fibres non-rigides.

Les compositions de mascara, selon l'invention, assurent un parfait allongement et un effet esthétique optimal, même dans le cas des cils fournis et/ou longs et/ou recourbés, pour lesquels des effets particulièrement inesthétiques étaient obtenus avec les compositions de l'art antérieur, comprenant des fibres, se courbant, non rigides.

Les compositions de mascara, selon l'invention, réalisent une continuité parfaite du cil, si bien que les fibres ne peuvent plus être observées à l'oeil nu car elles sont dans le prolongement exact du cil et « miment » parfaitement celui-ci.

Par « fibre », il faut comprendre un objet de longueur L et de diamètre D, tel que L soit généralement très supérieur à D, D étant le diamètre du cercle dans lequel s'inscrit la section de la fibre. En particulier, le rapport L/D (ou facteur de forme) est choisi dans la gamme allant de 3,5 à 2 500, de préférence de 5 à 500, et mieux de 5 à 150.

Les fibres utilisables dans la composition de l'invention peuvent être unitaires ou organisées, par exemple tressées, creuses ou pleines. Leur forme, à savoir la forme de leur section, peut être quelconque et notamment la section peut être circulaire ou polygonale, par exemple, triangulaire, carrée, hexagonale ou octogonale plate ou présenter plusieurs lobes (forme en « trèfle »), selon l'application spécifique envisagée. Généralement, leurs extrémités sont épointées et/ou polies pour éviter de se blesser. En outre, les fibres mises en oeuvre dans la composition de l'invention ne sont pas des fibres, par exemple des fibres plates, à structure multicouche de polymère (comportant au moins deux couches) telle que la structure multicouche crée un effet de couleur par interférences des rayons lumineux, qui diffractent et diffusent différemment selon les couches ; de telles fibres sont décrites dans les documents EP-A-911 217, EP-A-686 858 et US-A-5,472,798.

Généralement, les fibres ont une longueur allant de 0,8 à 5 mm, de préférence de 1 à 4 mm et mieux allant de 1 mm à 3 mm par exemple de 2 mm. Leur section peut être comprise dans un cercle d'un diamètre allant de 2 nm à 500 µm, de préférence allant de 100 nm à 100 µm et mieux de 1 µm à 50 µm.

Le poids ou titre des fibres est souvent donné en denier et représente le poids en gramme pour 9 km de fil. De préférence, les fibres selon l'invention ont un titre choisi dans la gamme allant de 0,15 à 30 deniers et mieux de 0,18 à 18 deniers.

Les fibres peuvent être présentes dans la composition selon l'invention en une teneur allant de 0,01 % à 10 % en poids, par rapport au poids total de la composition, de préférence de 0,1 % à 5 % en poids, et mieux de 0,3 % à 3 % en poids.

Les fibres sont selon l'invention choisies parmi les fibres de polyamide non aromatiques, tel que certains NYLON® , de polymère acrylique, de polyoléfine et notamment de polyéthylène ou de polypropylène, de polyesters, de polyuréthanes, et de polyimides-amides aromatiques, telles que les fibres KERMEL® et KERMEL TECH® de chez RHODIA, les fibres formées d'un mélange de polymères, tels que ceux mentionnés ci-avant, comme des fibres de polyamide/polyester.

Comme exemple de fibres rigides, on peut citer :

- des fibres rigides de polyesters, telles que celles obtenues par découpe de fils vendus sous les dénominations FIBRE 255-100-R11-242T TAILLE 3 MM (section octalobée), FIBRE 265-34-R11-56T TAILLE 3 MM (section ronde), FIBRE COOLMAX 50-34-591 TAILLE 3 MM (section tétralobée) par la société DUPONT DE NEMOURS ;
- des fibres rigides de polyamides, telles que celles vendues sous les dénominations TRILOBAL NYLON 0.120-1.8 DPF ; TRILOBAL NYLON 0.120-18 DPF ; NYLON 0.120-6 DPF par la société Cellusuede products ; ou obtenues par découpe de fils vendus sous la dénomination FIBRE NOMEX BRAND 430 TAILLE 3 MM par la société DUPONT DE NEMOURS.

Par ailleurs, les fibres peuvent être traitées ou non en surface et/ou enrobées ou non, par exemple par un agent actif et/ou un colorant et/ou un pigment.

L'enrobage, le dépôt, par exemple, d'agent actif, et/ou pigment et/ou colorant, sur les fibres peuvent se faire en plongeant les fibres dans une solution d'agent actif, afin de greffer ou d'adsorber l'agent actif à la surface des fibres.

On peut aussi incorporer un agent actif, et/ou pigment et/ou colorant dans la masse même du polymère dans la matrice du polymère formant les fibres. La présence de l'agent actif au sein même de la fibre permet d'améliorer sa stabilité, notamment s'il s'agit d'un agent actif sensible aux dégradations de toutes sortes.

En incorporant l'agent actif dans ou sur les fibres, on obtient une diffusion plus importante de l'agent actif sur une durée plus longue car les fibres restent à la surface de la peau et permettent la diffusion optimale de l'agent actif, tandis que lorsque celui-ci est mis en oeuvre isolément, sans être incorporé dans une fibre, il est attaqué et éliminé par les fluides corporels, tels que le sébum, les larmes, etc.

En d'autres termes, l'inclusion de l'agent actif et/ou pigment et/ou colorant dans la fibre provoque un effet que l'on peut qualifier généralement d'effet de rémanence.

Les produits KERMEL® et KERMEL TECH®, par exemple, sont teints dans la masse, ce qui offre une très grande durabilité des coloris dans le temps.

Avantageusement, on peut utiliser des fibres insolubles dans l'eau.

Cependant, les fibres particulièrement préférées, qui peuvent être incorporées dans les compositions de l'invention, sont les fibres de polyimides-amides ou polyamides-imides aromatiques, telles que les fibres KERMEL® et KERMEL TECH® de chez RHODIA®.

L'incorporation de ces fibres très spécifiques de polyimides-amides aromatiques dans des compositions cosmétiques, telles que des compositions de mascara permet d'obtenir tous les effets bénéfiques, mentionnés plus haut, en particulier en ce qui concerne les effets d'allongement et de « mime » du cil.

Bien évidemment, ces fibres de polyamides-imides répondent à la condition de rigidité, de courbure, à laquelle doivent satisfaire toutes les fibres mises en oeuvre selon l'invention.

Ces fibres ont une bonne inocuité cosmétique ; une bonne résistance chimique aux solvants et une bonne résistance mécanique.

Les polyimides-amides aromatiques entrant dans la composition des fibres selon l'invention peuvent être tout polyimide-amide aromatique, mais ils comprennent généralement un motif récurrent répondant à la formule générale (I) suivante :

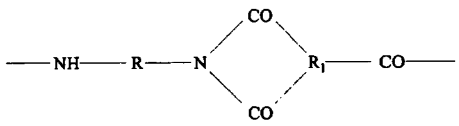

(I)

Ces polyimides-amides aromatiques comprennent, en outre, éventuellement, un motif récurrent (motif « amide ») de formule (II) :

- NH-R-NH-CO-$R_2$-CO- (II)

Ces polyimides-amides aromatiques comprennent, en outre, éventuellement, un motif récurrent (motif « amide ») de formule (III) :

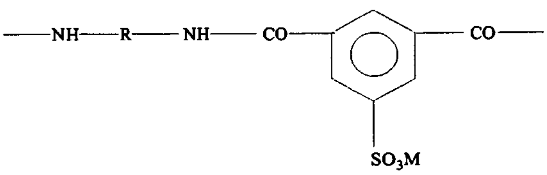

(III)

Ces polyimides-amides aromatiques comprennent, en outre, éventuellement, un motif récurrent (motif « amide ») de formule (IV) :

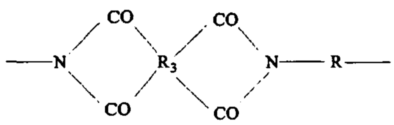

dans lesquelles R représente un groupe aromatique divalent, $R_2$ représente un groupe aromatique divalent, $R_3$ représente un groupe aromatique tétravalent, $R_1$ représente un groupe aromatique trivalent, et M représente un métal alcalin ou alcalino-terreux.

Par exemple, R et $R_2$ représentent chacun indépendamment un groupe divalent comprenant au moins un cycle aromatique, éventuellement substitué, ayant de 6 à 10 atomes de carbone et/ou un hétérocycle à caractère aromatique, éventuellement substitué, ayant de 5 à 10 atomes et comprenant un ou plusieurs hétéroatomes choisis parmi S, N et 0 ;

$R_1$ représente un groupe trivalent comprenant au moins un cycle aromatique carboné, éventuellement substitué, ayant de 6 à 10 atomes de carbone et/ou un hétérocycle à caractère aromatique, éventuellement substitué, ayant de 5 à 10 atomes et comprenant un ou plusieurs hétéroatomes choisis parmi S, N et 0.

Dans la formule (I) citée ci-dessus, $R_1$ peut être, par exemple, un cycle benzénique éventuellement substitué, par un ou deux substituant(s) choisi(s) parmi les groupes alkyle et alcoxy de 1 à 10 C, les atomes d'halogène, le groupe

nitro et le groupe sulfonyle ; ou plusieurs cycles benzéniques éventuellement substitués par un ou plusieurs substituant (s) choisi(s) parmi les groupes alkyle et alcoxy de 1 à 10 C, les atomes d'halogène, le groupe nitro et le groupe sulfonyle ; par exemple $R_1$ pourra comprendre de 2 à 5 cycles, reliés entre eux par une simple liaison ou par un groupe divalent, l'enchaînement desdits cycles pouvant être indépendamment en méta ou en para.

**[0061]** Ledit groupe divalent reliant lesdits cycles est choisi par exemple parmi :

- un groupe divalent dérivé d'un groupe alkyle linéaire ou ramifié (par exemple un groupe alkylidène ou alkylène) de 1 à 10 C éventuellement substitué, de préférence sur le même carbone, par un ou plusieurs halogènes choisis parmi F, Cl, Br et I et/ou par un ou plusieurs groupes hydroxyle(s), de préférence encore ledit groupe divalent est un groupe divalent dérivé d'un groupe alkyle perfluoré, par exemple alkylène perfluoré ;
- un hétéroatome choisi parmi O, S ;
- un groupe

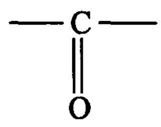 ;

un groupe

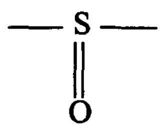 ;

un groupe

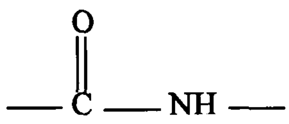 ,

un groupe

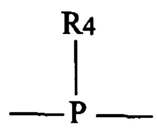 ;

un groupe

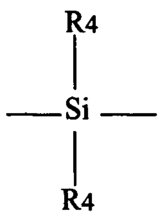 ;

un groupe

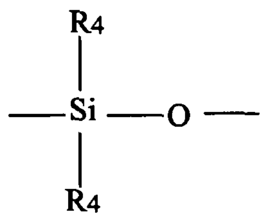

;

où $R_4$ est choisi parmi les groupes alkyle de 1 à 10 C tels que méthyle, éthyle, isopropyle, etc.

$R_1$ peut également représenter un groupe carboné polycyclique condensé éventuellement substitué par un ou plusieurs substituant(s) choisi(s) parmi les groupes alkyle et les groupes alcoxy de 1 à 10 C, les atomes d'halogène, le groupe nitro et le groupe sulfonyle, ledit groupe carboné polycyclique comprenant par exemple de 2 à 5 cycles benzéniques choisis par exemple parmi le naphtalène, le phénanthrène, le coronène, le pérylène, le phénylindane, etc.

$R_1$ peut aussi représenter un hétérocycle ou un hétérocycle condensé, à caractère aromatique tel que thiophène, pyrazine, pyridine, furanne, quinoléine, quinoxaline, isobenzofuranne, cet hétérocycle étant éventuellement substitué par un ou plusieurs substituants choisis parmi les groupes alkyles (par exemple méthyle, éthyle, isopropyle, etc.) et alcoxy de 1 à 10 C, les atomes d'halogènes (F, Cl, Br, I), le groupe nitro et le groupe sulfonyle.

Parmi les polyimides-amides utilisables dans le cadre de l'invention, on citera ceux dans lesquels $R_1$ est un cycle benzénique, un ensemble de deux cycles benzéniques reliés entre eux par un pont oxygène, ou un cycle naphtalénique.

Les groupes $R_1$ préférés sont :

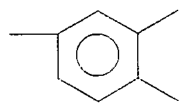

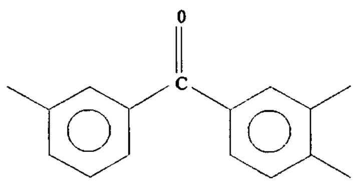

et

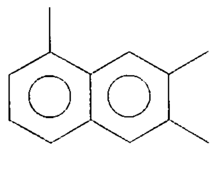

Le groupe $R_3$ répond à la même définition que le groupe $R_1$ à la différence qu'il s'agit d'un groupe tétravalent et non trivalent.

R et $R_2$ qui peuvent être identiques ou différents représentent chacun par exemple un cycle benzénique divalent à enchaînement meta, ou para ; éventuellement substitué par un ou plusieurs substituants choisis parmi les groupes alkyle et alcoxy de 1 à 10 C tels que méthyle, éthyle, isopropyle, butyle, méthoxy...., les atomes d'halogène, le groupe nitro et le groupe sulfonyle ; ou plusieurs cycles benzéniques éventuellement substitués par un ou plusieurs substituants choisis parmi les groupes alkyles et alcoxy de 1 à 10C, les atomes d'halogène, le groupe nitro, et le groupe sulfonyle,

par exemple R et $R_2$ peuvent comprendre de 2 à 5 cycles, reliés entre eux par une simple liaison ou par un groupe divalent.

Ledit groupe divalent reliant les cycles benzéniques de R ou $R_2$ est choisi par exemple parmi :

- un groupe divalent dérivé d'un groupe alkyle linéaire ou ramifié (par exemple un groupe alkylidène ou alkylène) de 1 à 10 C éventuellement substitué, de préférence sur le même carbone par un ou plusieurs halogènes choisis parmi F, Cl, Br et I et/ou par un ou plusieurs groupes hydroxyle(s), de préférence encore ledit groupe divalent est un groupe divalent dérivé d'un groupe alkyle perfluoré, par exemple alkylène perfluoré ;
- un hétéroatome choisi parmi O, S ;
- un groupe

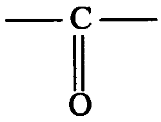

;

un groupe

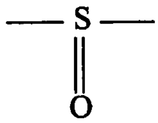

;

un groupe

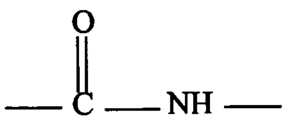

,

un groupe

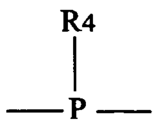

;

un groupe

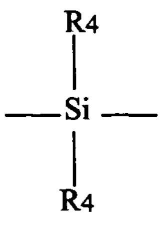

;

un groupe

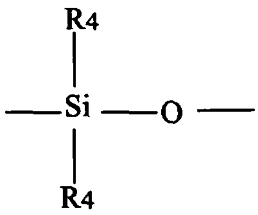

;

où $R_4$ est choisi parmi les groupes alkyle de 1 à 10 C tels que méthyle, éthyle, isopropyle, etc.

R et $R_2$ peuvent également chacun représenter un groupe carboné polycyclique condensé divalent éventuellement substitué par un ou plusieurs substituant(s) choisi(s) parmi les groupes alkyle et alcoxy de 1 à 10 C, les atomes d'halogène, le groupe nitro et le groupe sulfonyle, ledit groupe carboné polycyclique peut comprendre par exemple de 2 à 5 cycles benzéniques, et il peut être choisi par exemple parmi le naphtalène, le phénanthrène, le coronène, le pérylène, le phénylindane, etc.

$R_2$ peut aussi représenter un hétérocycle ou un hétérocycle condensé à caractère aromatique par exemple thiophène, pyrazine, pyridine, furanne, quinoléine, quinoxaline, isobenzofuranne, cet hétérocycle étant éventuellement substitué par un ou plusieurs substituant(s) choisi(s) parmi les groupes alkyle, et alcoxy de 1 à 10 C, par exemple méthyle, éthyle, isopropyle, méthoxy, les atomes d'halogènes (F, Cl, Br, I), le groupe nitro, et le groupe sulfonyle.

Les polyimides-amides préférées sont ceux dans lesquels R est un groupe diphénylméthane et $R_2$, un groupe phényl-1,4-diyle ; ou R un groupe diphényléther, et $R_2$ est un groupe phényl-1,4-diyle.

Des exemples du groupe $R_1$ ont déjà été donnés plus haut :

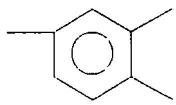

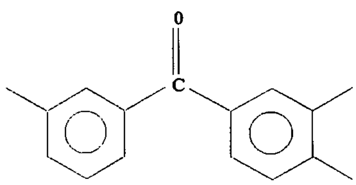

et

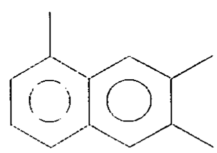

Des exemples du groupe $R_3$ sont les suivants :

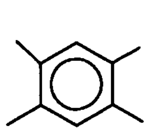 ; 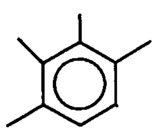 ;

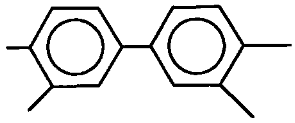 ; 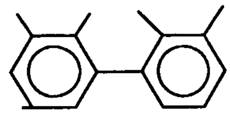 ;

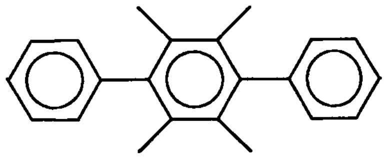 ; 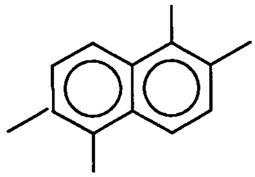 ;

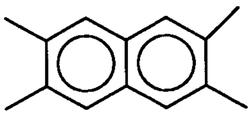 ; 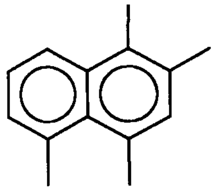 ; 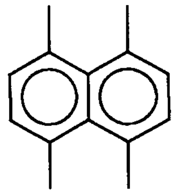 ;

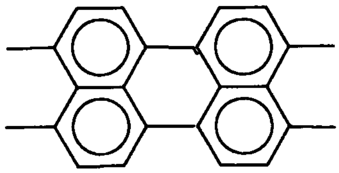 ; 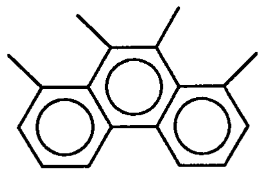 ;

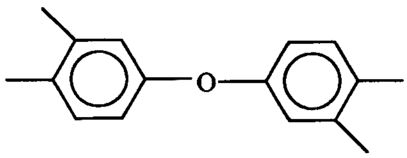 ; 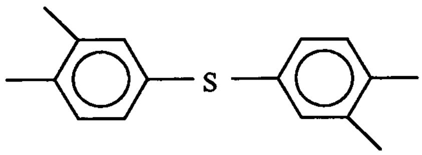

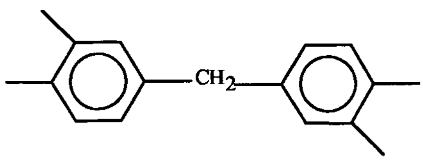 ; 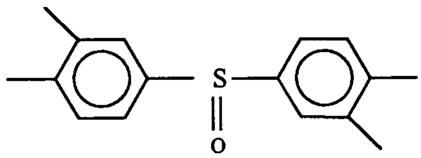 ;

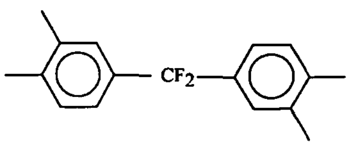 ; 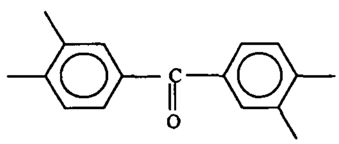 ;

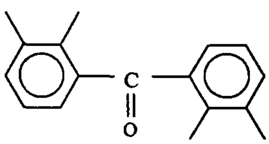 ; 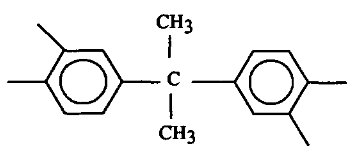 ;

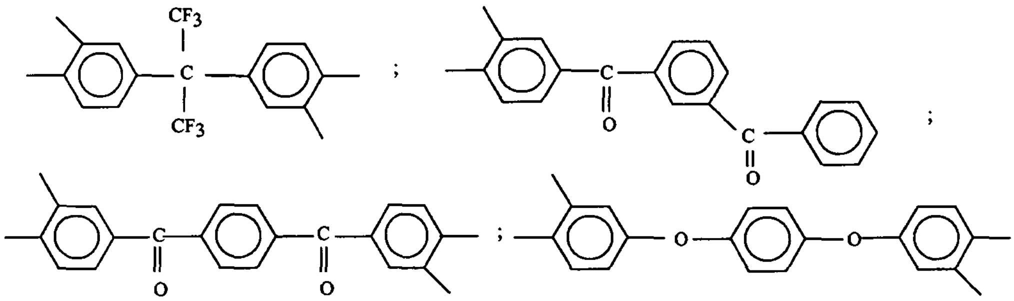

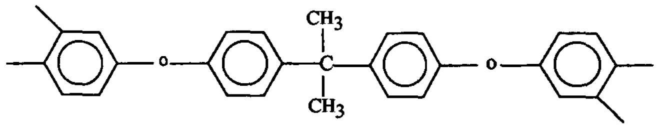

;

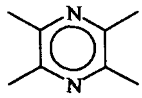

;

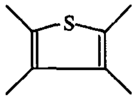

;

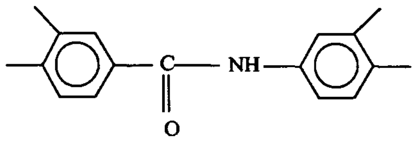

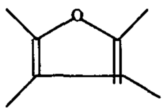

Il est à noter que d'autres exemples de groupes $R_1$ sont les équivalents trivalents des groupes tétravalents $R_3$ exemplifiés ci-dessus.

Des exemples des groupes R et $R_2$ sont les suivants :

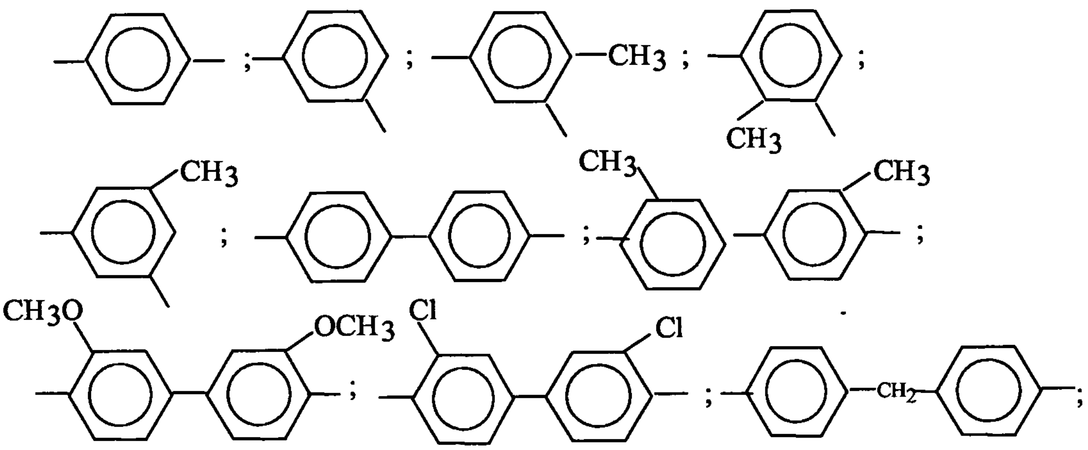

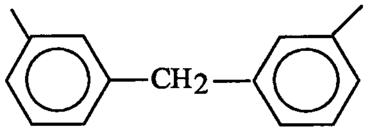 ; 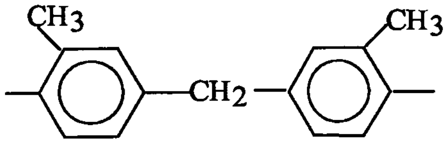 ;

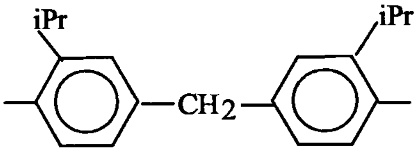 ; 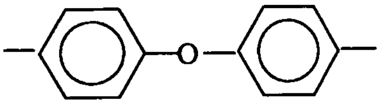 ;

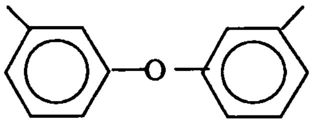 ; 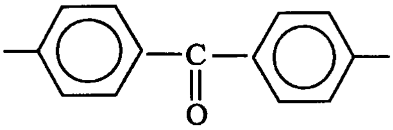 ;

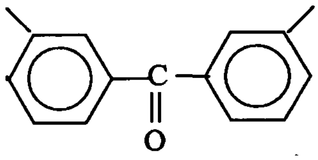 ; 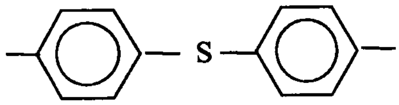 ;

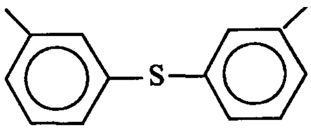 ; 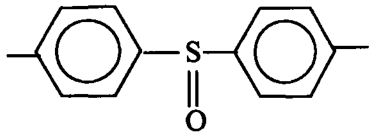 ;

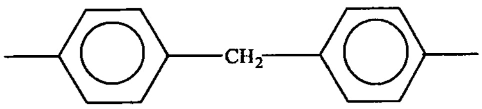 ; 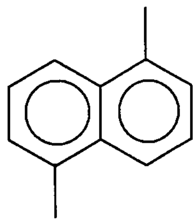

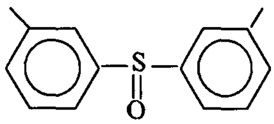 ; 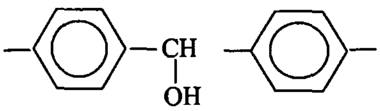 ;

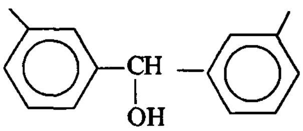 ; 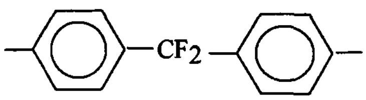 ;

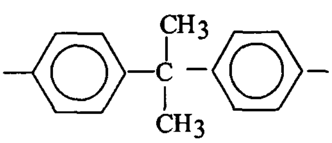 ; 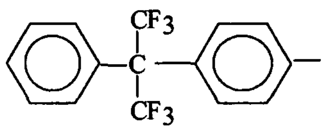 ; 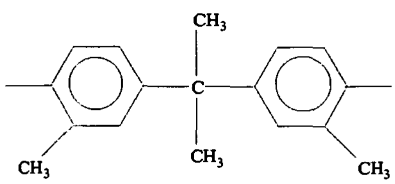

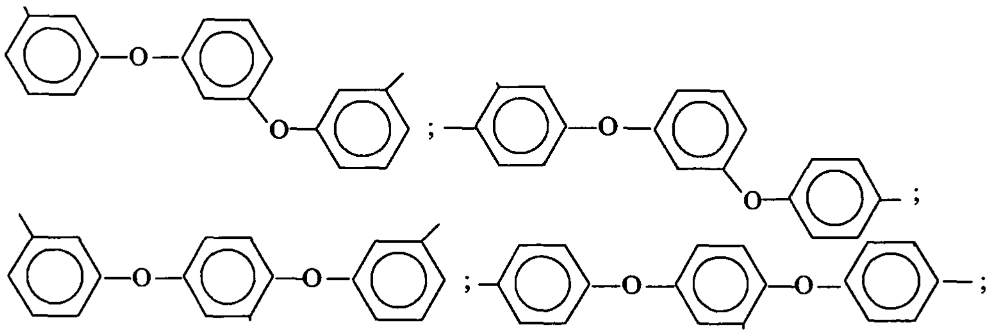

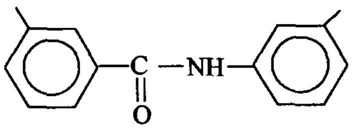 ; 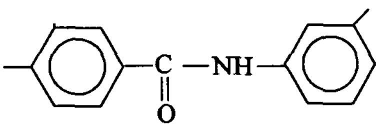 ;

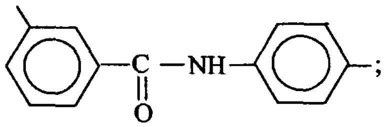 ; 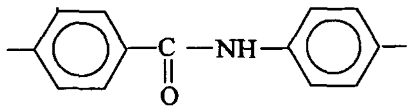 ;

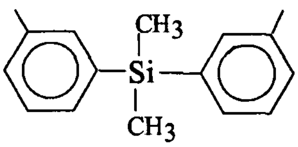 ; 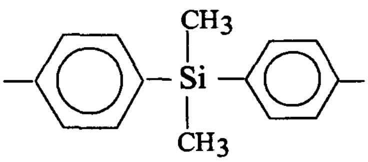 ; 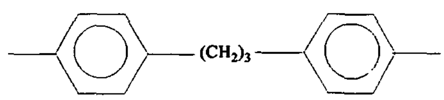

Les polyimides-amides aromatiques formant les fibres mises en oeuvre dans la composition de l'invention peuvent être obtenus par tous les procédés connus de l'homme du métier pour la préparation des polyimides-amides aromatiques, de préférence, par réaction d'un diisocyanate avec l'anhydride triméllitique.

Des procédés de préparation sont décrits, notamment dans les documents de PIGEON et le document US-A-3 802 841, cités ci-après.

Des fils ou fibres de polyimide-amide, qui peuvent être utilisés pour les compositions de l'invention, sont décrits, par exemple, dans le document de R. PIGEON et P. ALLARD, Chimie Macromoléculaire Appliquée, 40/41 (1974), pages 139-158 (n° 600), auquel on pourra se référer. Le polyamide-imide est préparé par polycondensation directe de l'anhydride triméllitique avec un diisocyanate aromatique de formule :

O=C-N-R-N-C=O

par élimination du $CO_2$.

Le diisocyanate aromatique peut être remplacé par son dérivé uréthane de formule :

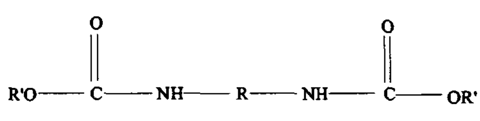

Dans ces formules, R, qui peut avoir la signification déjà donnée plus haut, représente, de préférence :

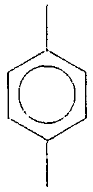 ; 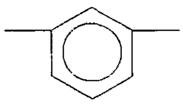 ; 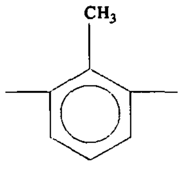 ; 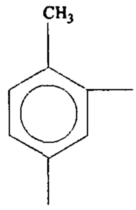 ;

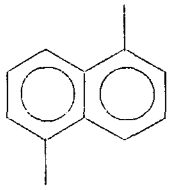 ; 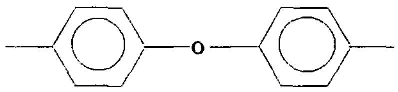 ; 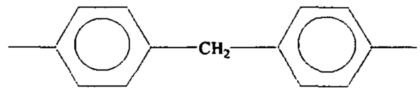 ;

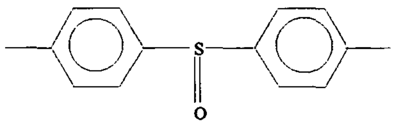 ; 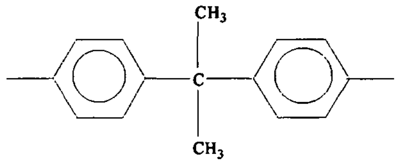 ;

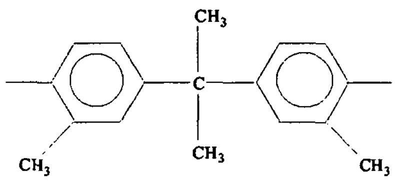 .

En outre, on peut aussi obtenir un copolyimide-imide en remplaçant partiellement l'anhydride triméllitique (TMA) par un acide dicarboxylique ou un dianhydride (troisième composant), ce qui provoque une modification du rapport amide/amide dans le polymère. Le troisième composant peut être choisi parmi l'acide isophtalique, l'acide téréphtalique, l'acide diphénylique, l'acide dicarboxylique de phénylindane, et l'anhydride pyromellitique. Ce troisième composant peut être présent dans une proportion pouvant aller jusqu'à 10, 20, 30 ou 40 % en moles du mélange TMA et troisième composant.

La réaction de l'anhydride triméllitique, avec un diisocyanate, s'effectue dans un solvant polaire, tel que le DMF, le DMAC, le NMP, et leurs mélanges. Les solvants et réactifs sont, de préférence, d'une grande pureté.

Les réactifs, en quantité stoechiométrique, sont ajoutés sous agitation dans un réacteur, chauffé selon un programme de chauffage déterminé et on arrête la réaction par refroidissement, quand la viscosité correcte est obtenue.

Par exemple, la réaction, notamment pour obtenir les amides de triméllitide de polydiphényl méthane peut être réalisée par chauffage à de 80 à 150°C, pendant 3,5 heures, puis chauffage à 450°C, pendant 4,5 heures, ou bien par chauffage pendant 8 heures, à de 80°C, à 150°C, par exemple de 80, 120, ou 150°C.

Après réglage de la concentration et filtration, la solution est filée selon les procédés connus par voie humide et à sec et les fils sont coupés à la longueur de fibre voulue pour être incorporées dans les compositions selon l'invention.

Le filage se fait, sans difficultés particulières, aussi bien par un procédé à sec que par un procédé par voie humide, à condition qu'il y ait un rapport correct entre la viscosité et la concentration.

Dans le filage à sec, la solution est comprimée à l'aide d'une pompe de titrage par le biais d'une buse de filage qui se situe à l'extrémité supérieure d'un réservoir chauffé à la verticale. Le solvant s'évapore sous l'action du flux de gaz et est à nouveau piégé par condensation.

Dans le filage par voie humide, la buse de filage est plongée dans un bain précipité d'eau et de solvant. Dans ce bain, le polymère est précipité sous forme de gel de monofils qui sont ensuite partiellement étirés, lavés et séchés. Il faut cependant souligner que pour enlever complètement le solvant, un traitement de lavage plus en profondeur que pour les fibres traditionnelles filées à partir d'une solution doit être réalisé. On peut ajouter à la solution de filage, sans problème, d'autres additifs, notamment des pigments. De cette manière, on peut obtenir une série de coloris dotés d'une

bonne tenue, aussi bien pour les fils sans fin que les fils de fibres.

D'autres polyimide-amides, qui peuvent être utilisés pour former les fibres des compositions de l'invention, sont décrits dans le document US-A-3 802 841, à la description duquel on pourra également se reporter.

Ce document US décrit la préparation de polyimides-amides par réaction d'une diamine ou de l'un de ses dérivés, tel qu'un diisocyanate aromatique avec un anhydride d'acide ou l'un de ses dérivés.

Les diisocyanates aromatiques sont, par exemple, le toluylène diisocyanate, le 4',4'-diisocyanatodiphénylméthane, le 4',4'-diisocyanatodiphénylpropane et le 4'-diisocyanatodiphényléther. Ainsi, dans ce document, on fait réagir un acide polycarboxylique, de préférence, un acide tricarboxylique avec une ou plusieurs diamines. Les acides polycarboxyliques, qui sont utilisés avantageusement dans la préparation du polyamine-imide, sont, par exemple, l'acide triméllitique, l'acide benzophénone 3,3',4'-tricarboxylique et l'acide naphtalène-1,4,5-tricarboxylique ou des dérivés de celles-ci.

En outre, un diacide ou un dérivé de celle-ci, tel qu'un chlorure d'acide, peut être utilisé dans le système réactionnel pour créer la liaison amide-imide, des exemples de ces acides sont l'acide isophtalique, et l'acide téréphtalique, éventuellement substitués. D'autres acides dicarboxyliques et chlorure de ceux-ci sont cités à la colonne 3, ligne 32, colonne 4, ligne 42 de ce document, auxquelles on pourra se reporter.

Les diamines sont citées à la colonne 1, ligne 44 - colonne 2, ligne 51 de ce document, auxquelles on pourra se reporter.

Un exemple de polyimide-amide, préparé dans ce document, est obtenu par réaction de 100 moles de 4,4'-diisocyanatodiphénylméthane avec 80 moles d'anhydride triméllitique, 16 moles d'acide téréphtalique et 4 moles de sel de sodium de l'acide sulfo-5-isophtalique.

D'autres polyimides-amides aromatiques, qui peuvent être utilisés dans les compositions de l'invention, font l'objet du document FR-A-2 079 785, qui décrit des fils brillants à base de polyimide-amide contenant au moins 3 % de motifs, enchaînements issus de dicarboxy-3,5-benzène sulfonate alcalin ou alcalino-terreux, par filage humide d'une solution de polymère dans la N-méthylpyrrolidone, dans un bain coagulant aqueux contenant également de la N-méthylpyrrolidone, puis étirage, lavage et séchage.

Les compositions de l'invention peuvent aussi comprendre des fibres de polyimides-amides aromatiques dont le polymère est décrit dans le document EP-A1-0 360 728.

Ce polymère comprend :

- des motifs amide-imide de formule :

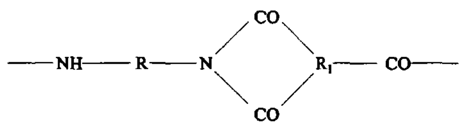

(I)

- des motifs amides de formule :

-NH-R-NH-CO-$R_2$-CO-

- et, éventuellement, des motifs amide de formule :

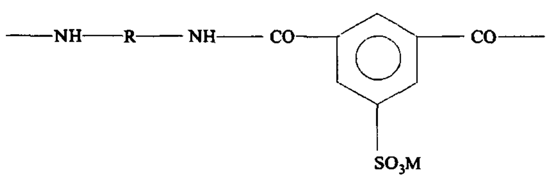

dans lesquelles, et comme déjà mentionné plus haut, R représente un radical aromatique divalent, $R_1$ représente un radical aromatique trivalent, $R_2$ représente un radical aromatique divalent et M est un métal alcalin ou alcalino-terreux.

De préférence, les motifs (I) représentent de 80 à 100 % des motifs, les motifs (II) représentent de 1 à 5 % des motifs et les motifs (III) représentent de 0 à 20 % de l'ensemble des motifs.

Les polyimides-amides (PIA), de ce document, sont obtenus par polycondensation dans tout solvant approprié :

- d'au moins un diisocyanate de formule :

OCN-R-NCO

dans laquelle R est un groupe aromatique divalent, déjà défini plus haut, tel que le diphénylméthane-4,4'-diisocyanate et, de préférence, le diphényléther-4,4'-diisocyanate ou leurs mélanges avec :

- un anhydrique acide aromatique, tel que l'anhydride triméllitique ;
- un diacide aromatique, tel que l'acide téréphtalique ;
- et, éventuellement, un dicarboxybenzène sulfonate de métal alcalin ou alcalino-terreux, de préférence le dicarboxybenzène sulfonate de sodium ou de potassium.

Les fils et fibres de ce document possèdent une structure chimique, telle que définie ci-dessus, dans laquelle R est un radical , tel que :

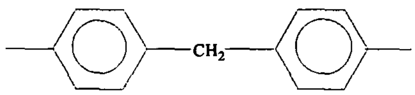

et, de préférence :

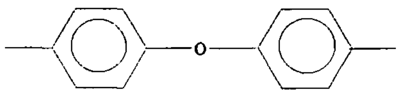

et $R_1$, de préférence, un radical :

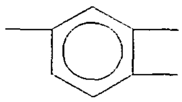

$R_2$, de préférence, un radical :

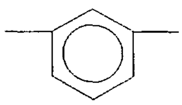

et M est, de préférence, Na ou K.

Les fils de PIA, selon ce document, sont obtenus par filage à l'humide, à partir de solutions de polymère dans un solvant ou mélange solvant. La concentration des solutions de filage est comprise entre 4 et 35 %, de préférence entre 5 et 35 %. Les polymères sont dissous dans un solvant ou mélange solvant contenant de 5 à 100 % en poids de diméthyléthylène urée de pH < 7 et 0 à 55 % d'un solvant polaire aprotique anhydre, tel que la N-méthylpyrrolidone, le diméthylacétamide, le diméthylformamide, la tétraméthylurée ou la y butyrolactone.

Les solutions utilisables doivent posséder une viscosité comprise entre 100 et 200 poises, de préférence entre 150 et 160 poises. Elles doivent également contenir divers adjuvants, tels que pigments, matifiants pour améliorer certaines propriétés.

Les solutions de PIA sont filées dans un bain coagulant aqueux binaire ou ternaire contenant un solvant ou mélange solvant, en proportion de 30 à 80 % de solvant et 20 à 70 % d'eau, de préférence 40 à 70 % de solvant(s).

Le solvant utilisé peut être le diméthylformamide, la diméthylèthylène urée ou leur mélange. Le bain de filage est maintenu entre 15 et 40°C, de préférence 20 à 30°C. La longueur du bain coagulant est adaptable en fonction généralement de la concentration en solvant et de la température. Des bains ayant une plus forte teneur en solvant

permettent d'obtenir des fils de meilleure étirabilité, donc de meilleures propriétés finales. Toutefois, lorsque la concentration en solvant est plus élevée, une plus grande longueur de bain est nécessaire. Les filaments sortant du bain coagulant à l'état de gel sont ensuite étirés, par exemple dans l'air à un taux défini par le rapport $\frac{V2}{V1} x 100$, V2 étant la vitesse des rouleaux d'étirage, et V1 celle des rouleaux délivreurs. Le taux d'étirage des fils à l'état de gel est d'au moins 100 %, de préférence au moins 110 % ou même plus.

Après étirage, les filaments sont lavés par des moyens connus pour les débarrasser du ou des solvants. Ce lavage peut être effectué, par exemple, dans des bacs successifs, dans lesquels de l'eau circule à contre-courant ou sur des rouleaux laveurs ou par tout autre moyen, de préférence à température ambiante.

Les filaments lavés sont alors séchés par des moyens connus, par exemple dans un séchoir ou sur des rouleaux. La température de ce séchage peut varier dans de grandes limites, ainsi que la vitesse qui est d'autant plus grande que la température est plus élevée. On a généralement avantage à effectuer un séchage avec élévation progressive de la température, cette température pouvant atteindre et même dépasser 200°C, par exemple.

Les filaments peuvent éventuellement subir ensuite un surétirage à chaud pour améliorer leurs qualités mécaniques et en particulier leur ténacité, ce qui peut être intéressant pour certains emplois.

Ce surétirage à chaud peut être effectué par tout moyen connu : four, plaque, rouleau, rouleau et plaque, de préférence dans une enceinte fermée. Il doit être effectué à température d'au moins 150°C, pouvant atteindre et même dépasser 200 à 300°C. Son taux est généralement d'au moins 150 %, mais il peut varier dans de grandes limites, selon les qualités désirées pour le fil fini. Le taux d'étirage total est d'au moins 250 %, de préférence au moins 260 %.

L'ensemble étirage et surétirage peut être effectué en un ou plusieurs stades, en continu ou en discontinu, avec les opérations précédentes. De plus, l'étirage secondaire peut être combiné avec le séchage. Il suffit pour cela de prévoir, à la fin du séchage, une zone de température plus élevée permettant le surétirage.

En vue d'être incorporés dans la composition de l'invention, les filaments sont ensuite découpés en fibres de la longueur voulue, mentionnée plus haut.

Un polymère faisant l'objet de ce document et qui peut former les fibres incluses dans les compositions de l'invention est préparé à partir des monomères suivants :

- anhydride triméllitique : 40 mole % ;
- acide isophtalique : 8 mole % ;
- sel de sodium de l'acide sulfo-5-isophtalique : 2 mole % ;
- diisocyanate-4,4'-diphényléther : 50 mole %.

Le document EP-A-0 549 494 décrit également des fibres à base de polyimides-amides qui peuvent être incorporées dans les compositions de l'invention. Il s'agit de fibres de PAI constituées :

- de motifs amides-imides de formule (I) ;
- éventuellement, de motifs de formule (II) ;
- éventuellement, de motifs de formule (III) ;
- de motifs de formule (IV).

R, $R_1$, $R_2$, $R_3$ et $R_4$ ont la signification déjà donnée plus haut. En particulier, R représente un radical tolylène et/ou métaphénylène. Les motifs (I) sont présents, à raison de 0 à 100 %, de préférence de 20 à 100 %, les motifs (II) sont présents, à raison de 0 à moins de 100 %, les motifs (III) sont présents, à raison de 0 à 5 % et les motifs (IV) sont présents à raison de 0 à moins de 100 %, de préférence de 0 à 80 %. La somme des motifs (I), (II), (III) et (IV) étant égale à 100 %.

Comme on l'a déjà indiqué plus haut, les fibres de polyimide-amide aromatique préférées sont des fibres de KERMEL TECH®, dans lesquelles le polyimide-amide comprend des motifs répétitif de formule :

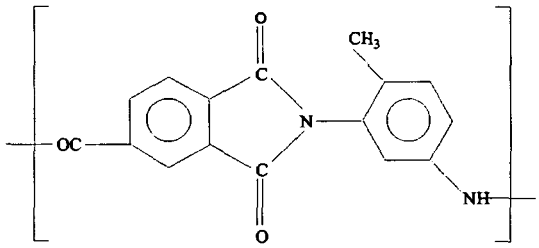

et est obtenu par polycondensation du toluylène diisocyanate et de l'anhydride triméllitique.

La composition, selon l'invention, est une composition de maquillage, une base de maquillage, une composition dite « top-coat » à appliquer sur un maquillage, ou une composition de traitement cosmétique, de soin, des matières, fibres, kératiniques.

La composition selon l'invention s'applique plus particulièrement aux cils. De ce fait, la composition de l'invention peut être une composition de revêtements des cils, notamment une composition de maquillage des cils, encore appelé mascara, une composition à appliquer sur un maquillage des cils, dite encore top-coat, ou bien encore une composition de traitement des cils, notamment des cils d'êtres humains ou des faux cils. Plus spécialement, la composition est un mascara.

Dans la présente demande, on entend par "milieu physiologiquement acceptable", un milieu non toxique compatible avec les matières kératiniques d'êtres humains, notamment les cils ou les sourcils, comme un milieu cosmétique, le milieu cosmétique pouvant être un milieu cosmétique hydrophile ou lipophile.

La composition selon l'invention peut comprendre un milieu aqueux, constituant une phase aqueuse, qui peut être la phase continue de la composition.

La composition peut comprendre de l'eau et éventuellement un ou plusieurs solvant (s) organique (s) hydrophile ( s ) , c' est-à-dire un ou des solvant (s) organique(s) miscibles à l'eau, comme les alcools et notamment des monoalcools ayant de 2 à 5 atomes de carbone comme l'éthanol, l'isopropanol ou le n-propanol, les polyols ayant de 2 à 8 atomes de carbone comme la glycérine, la diglycérine, le propylène glycol, l'éthylène glycol, le 1,3-butylène glycol, le sorbitol, le penthylène glycol, les cétones en $C_3$-$C_4$, les aldéhydes en $C_2$-$C_4$.

L'eau ou le mélange d'eau et de solvant(s) organique(s) hydrophile(s) peut être présent dans la composition selon l'invention en une teneur allant de 0,1 % à 90 % en poids, par rapport au poids total de la composition, et de préférence de 0,1 % à 60 % en poids.

La composition peut également comprendre une phase grasse, pouvant comprendre des corps gras choisis parmi les huiles, les solvants organiques, les cires, les corps gras pâteux, et leurs mélanges. La phase grasse peut former une phase continue de la composition. En particulier, la composition selon l'invention peut être anhydre.

La phase grasse peut notamment être constituée de toute huile physiologiquement acceptable et en particulier cosmétiquement acceptable, notamment choisie parmi les huiles d'origine minérale, animale, végétale ou synthétique, carbonées, hydrocarbonées, fluorées et/ou siliconées, seules ou en mélange.

La phase grasse totale de la composition peut représenter de 0,1 % à 98 % en poids, par rapport au poids total de la composition, et de préférence de 1 à 80 % en poids.

Avantageusement, la phase grasse de la composition peut comprendre au moins une huile ou solvant organique volatile et/ou au moins une huile non volatile. De préférence, encore la phase grasse comprend au moins une huile volatile.

Par "composé volatil", par exemple par "huile ou solvant organique volatil", on entend au sens de l'invention tout composé (ou milieu non aqueux) susceptible de s'évaporer au contact de la peau ou de la fibre, matière kératinique, en moins d'une heure, à température ambiante et pression atmosphérique. Le composé volatil, par exemple le ou les solvants organiques volatils et les huiles volatiles de l'invention est un composé cosmétique volatil (ce sont par exemple des solvants organiques et des huiles cosmétiques volatiles), liquide à température ambiante, ayant notamment une pression de vapeur non nulle, à température ambiante et pression atmosphérique, allant de $10^{-3}$ à 300 mm de Hg (0,13 Pa à 40 000 Pa), plus particulièrement allant de 1,3 Pa à 13 000 Pa (0, 01 à 100 mm de Hg), et encore plus particulièrement allant de 1,3 Pa à 1300 Pa (0, 01 à 10 mm de Hg) . Par opposition, on entend par "composé non volatil", par exemple par "huile non volatile", un composé par exemple une huile restant sur la peau ou la fibre, matière kératinique à température ambiante et pression atmosphérique au moins plusieurs heures et ayant notamment une pression de vapeur inférieure à $10^{-3}$ mm de Hg (0,13Pa).

Ces huiles peuvent être des huiles hydrocarbonées, des huiles siliconées, des huiles fluorées, ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles

peuvent être choisies parmi les huiles hydrocarbonées ayant de 8 à 16 atomes de carbones, et notamment les alcanes ramifiés en $C_8$-$C_{16}$ comme les isoalcanes en $C_8$-$C_{16}$ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars' ou de Permetyls, les esters ramifiés en $C_8$-$C_{16}$ le néopentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées. De préférence, le solvant volatil est choisi parmi les huiles volatiles hydrocarbonées ayant de 8 à 16 atomes de carbone et leurs mélanges.

Comme huiles volatiles, on peut aussi utiliser les silicones volatiles, comme par exemple les huiles de silicones linéaires ou cycliques volatiles, notamment celles ayant une viscosité ≤ 6 centistokes (6 $10^{-6}$ $m^2/s$), et ayant notamment de 2 à 10 atomes de silicium, ces silicones comportant éventuellement des groupes alkyle ou alkoxy ayant de 1 à 22 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

On peut également utiliser des solvants volatils fluorés tels que le nonafluorométhoxybutane ou le perfluorométhylcyclopentane.

L'huile volatile peut être présente dans la composition selon l'invention en une teneur allant de 0,1 % à 98 % en poids, par rapport au poids total de la composition, de préférence de 1 % à 65 % en poids.

La composition peut également comprendre au moins une huile non volatile, et notamment choisie parmi les huiles hydrocarbonées et/ou siliconées et/ou fluorées non volatiles.

Comme huile hydrocarbonée non volatile, on peut notamment citer :

- les huiles hydrocarbonées d'origine végétale telles que les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_5 + R_6$ soit ≥ 10, comme par exemple l'huile de Purcellin (octanoate de cétostéaryle), le myristate d'isopropyle, le palmitate d'isopropyle, le benzoate d'alcool en $C_{12}$ à $C_{15}$, le laurate d'hexyle, l'adipate de diisopropyle, l'isononanoate d'isononyle, le palmitate de 2-éthylhexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;
- et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles fluorées utilisables dans l'invention sont notamment des huiles fluorosiliconées, des polyéthers fluorés, des silicones fluorées telles que décrit dans le document EP-A-847752.

Les huiles non volatiles peuvent être présentes dans la composition selon l'invention en une teneur allant de 0,1 à 80% en poids, de préférence de 0,1% à 50% en poids, par rapport au poids total de la composition, et mieux de 0,1% à 20% en poids.

La phase grasse de la composition selon l'invention peut comprendre une cire. Par "cire", on entend au sens de la présente invention, un composé gras lipophile, solide à température ambiante (25°C) et pression atmosphérique (760 mm de Hg, soit 105 Pa), à changement d'état solide/liquide réversible, ayant une température de fusion supérieure à 30°C et mieux supérieure à 55 °C et pouvant aller jusqu'à 200°C, notamment jusqu'à 120°C.

En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les valeurs de point de fusion correspondent, selon l'invention, au pic de fusion mesurée à l'aide d'un calorimètre à balayage différentiel (D.S.C.), par exemple le calorimètre vendu sous la dénomination DSC 30 par la société METLER , avec une montée en température de 5 ou 10 °C par minute.

Les cires, au sens de l'invention, sont celles généralement utilisées dans les domaines cosmétique et dermatologique . On peut notamment citer la cire d'abeilles, la cire de lanoline, et les cires d'insectes de Chine; la cire de riz, la cire de Carnauba, la cire de Candellila, la cire d'Ouricury, la cire de fibres de liège, la cire de canne à sucre, la cire du Japon et la cire de sumac; la cire de montan, les cires microcristallines, les cires de paraffine, les ozokérites, la cire de cérésine, la cire de lignite, les cires de polyéthylène, les cires obtenues par la synthèse de Fisher-Tropsch, les esters d'acides gras et les glycérides concrets à 40°C et mieux à plus de 55°C.

On peut aussi citer les cires obtenues par hydrogénation catalytique d'huiles animales ou végétales ayant des chaînes grasses, linéaires ou ramifiées, en C8-C32. Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée.

On peut encore citer les cires de silicone ou les cires fluorées.

Les cires présentes dans la composition peuvent être dispersées sous forme de particules dans un milieu aqueux. Ces particules peuvent avoir une taille moyenne allant de 50 nm à 10 μm, et de préférence de 50 nm à 3,5 μm.

En particulier, la cire peut être présente sous forme d'émulsion cires-dans-eau, les cires pouvant être sous forme de particules de taille moyenne allant de 1 μm à 10 μm, et de préférence de 1 μm à 3,5 μm.

Dans un autre mode de réalisation de la composition selon l'invention, la cire peut être présente sous forme de microdispersion de cire, la cire étant sous forme de particules dont la taille moyenne est inférieure à 1 μm, et va notamment de 50 nm à 500 nm. Des microdispersions de cires sont décrites dans les documents EP-A-557196, EP-A-1048282.

La cire peut également présenter une dureté allant de 0,05 MPa à 15 MPa, et de préférence allant de 6 MPa à 15 MPa . La dureté est déterminée par la mesure de la force en compression mesurée à 20°C à l'aide du texturomètre vendu sous la dénomination TA-XT2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm. Pour effectuer la mesure de dureté, la cire est fondue à une température égale au point de fusion de la cire + 20°C. La cire fondue est coulée dans un récipient de 30 mm de diamètre et de 20 mm de profondeur. La cire est recristallisée à température ambiante (25°C) pendant 24 heures, puis la cire est conservée pendant au moins 1 heure à 20°C avant d'effectuer la mesure de dureté. La valeur de la dureté est la force de compression mesurée divisée par la surface du cylindre du texturomètre en contact avec la cire.

La cire peut être présente dans la composition selon l'invention en une teneur allant de 0,1% à 50% en poids, par rapport au poids total de la composition, de préférence de 0,5% à 30% en poids, et mieux de 1% à 20% en poids.

La composition selon l'invention peut comprendre au moins un composé gras pâteux à température ambiante. Par "corps gras pâteux" au sens de l'invention, on entend des corps gras ayant un point de fusion allant de 20 à 55°C, de préférence 25 à 45°C, et/ou une viscosité à 40°C allant de 0,1 à 40 Pa.s (1 à 400 poises), de préférence 0,5 à 25 Pa.s, mesurée au Contraves TV ou Rhéomat 80, équipé d'un mobile tournant à 60 Hz. L'homme du métier peut choisir le mobile permettant de mesurer la viscosité, parmi les mobiles MS-r3 et MS-r4, sur la base de ses connaissances générales, de manière à pouvoir réaliser la mesure du composé pâteux testé.

De préférence, ces corps gras sont des composés hydrocarbonés, éventuellement de type polymérique ; ils peuvent également être choisis parmi les composés siliconés et/ou fluorés ; ils peuvent aussi se présenter sous forme d'un mélange de composés hydrocarbonés et/ou siliconés et/ou fluorés. Dans le cas d'un mélange de différents corps gras pâteux, on utilise de préférence les composés pâteux hydrocarbonés (contenant principalement des atomes de carbone et d'hydrogène et éventuellement des groupements ester), en proportion majoritaire.

Parmi les composés pâteux susceptibles d'être utilisés dans la composition selon l'invention, on peut citer les lanolines et les dérivés de lanoline comme les lanolines acétylées ou les lanolines oxypropylènées ou le lanolate d'isopropyle, ayant une viscosité de 18 à 21 Pa.s, de préférence 19 à 20,5 Pa.s, et/ou un point de fusion de 30 à 55°C et leurs mélanges. On peut également utiliser des esters d'acides ou d'alcools gras, notamment ceux ayant 20 à 65 atomes de carbone (point de fusion de l'ordre de 20 à 35°C et/ou viscosité à 40°C allant de 0,1 à 40 Pa.s) comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux comme l'acide poly(12-hydroxystéarique) et leurs mélanges. Comme triglycérides d'origine végétale, on peut utiliser les dérivés d'huile de ricin hydrogénée, tels que le "THIXINR" de Rhéox.

On peut aussi citer les corps gras pâteux siliconés tels que les polydiméthylsiloxanes (PDMS) ayant des chaînes pendantes du type alkyle ou alcoxy ayant de 8 à 24 atomes de carbone, et un point de fusion de 20-55°C, comme les stearyl dimethicones notamment ceux vendus par la société Dow Corning sous les noms commerciaux de DC2503 et DC25514, et leurs mélanges.

Le corps gras pâteux peut être présent dans la composition selon l'invention en une teneur allant de 0 à 60% (notamment 0,01% à 60%) en poids, par rapport au poids total de la composition, de préférence allant de 0,5 à 45% en poids, et mieux allant de 2% à 30% en poids, dans la composition.

La composition selon l'invention peut contenir des agents tensioactifs émulsionnants présents notamment en une proportion allant de 2 à 30% en poids par rapport au poids total de la composition, et mieux de 5% à 15%. Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans la composition selon l'invention sont choisis :

- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de $C_1$-$C_6$ alkyl glucose polyoxyéthylénés, et leurs mélanges.
- parmi les tensioactifs anioniques : les acides gras en $C_{16}$-$C_{30}$ neutralisés par les amines, l'ammoniaque ou les sels alcalins, et leurs mélanges.

On utilise de préférence des tensioactifs permettant l'obtention d'émulsion huile-dans-eau ou cire-dans-eau.

La composition selon l'invention peut comprendre un polymère filmogène.

Le polymère filmogène peut être un polymère solubilisé ou dispersé sous forme de particules dans une phase aqueuse de la composition ou bien encore solubilisé ou dispersé sous forme de particules dans une phase grasse liquide. La composition peut comprendre un mélange de ces polymères.

Le polymère filmogène peut être présent dans la composition selon l'invention en une teneur en matières sèches allant de 0,1% à 60% en poids par rapport au poids total de la composition, de préférence de 0,5% à 40% en poids, et mieux de 1% à 30% en poids.

Dans la présente demande, on entend par "polymère filmogène", un polymère apte à former à lui seul ou en présence d'un agent auxiliaire de filmification, un film continu et adhérent sur un support, notamment sur les matières kératiniques.

On utilise de préférence un polymère filmogène apte à former un film hydrophobe, c'est-à-dire un polymère dont le film a une solubilité dans l'eau à 25 °C inférieure à 1 % en poids.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).

Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés $\alpha,\beta$-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth) acrylique (encore appelé les (méth) acrylates) , notamment des (méth)acrylates d'alkyle, en particulier d'alkyle en $C_1$-$C_{30}$, de préférence en $C_1$-$C_{20}$, des (méth)acrylates d'aryle, en particulier d'aryle en $C_6$-$C_{10}$, des (méth) acrylates d' hydroxyalkyle, en particulier d'hydroxyalkyle en $C_2$-$C_6$ .

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en $C_2$-$C_{12}$. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styrèniques, on peut citer le styrène et l'alpha-méthyl styrène.

Il est possible d'utiliser tout monomère connu de l'homme du métier entrant dans les catégories de monomères acryliques et vinyliques (y compris les monomères modifiés par une chaîne siliconée).

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les poly-uréthanes-polyvinylpirrolidones, les polyester-polyuréthanes, les polyéther-polyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.

L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexa-nedicarboxylique, l'acide isophtalique, l'acide téréphtalique, l'acide 2,5-norborane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol.

Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement $-SO_3M$, avec M représentant un atome d'hydrogène, un ion ammonium $NH_4^+$ ou un ion métallique, comme par exemple un ion $Na^+$, $Li^+$, K+, $Mg^{2+}$, $Ca^{2+}$, $Cu^{2+}$, $Fe^{2+}$ , $Fe^{3+}$. On peut utiliser notamment un monomère aromatique bifonctionnel comportant un tel groupement $-SO_3M$.

Le noyau aromatique du monomère aromatique bifonctionnel portant en outre un groupement $-SO_3M$ tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement $-SO_3M$ : l'acide sulfoisophtalique, l'acide sulfotéréphtalique, l'acide sulfophtalique, l'acide 4-sulfonaphtalène-2,7-dicarboxylique.

On préfère utiliser des copolymères à base d'isophtalate/sulfoisophtalate, et plus particulièrement des copolymères obtenus par condensation de di-éthylèneglycol, cyclohexane diméthanol, acide isophtalique, acide sulfoisophtalique. De tels polymères sont vendus par exemple sous le nom de marque Eastman AQ® par la société Eastman Chemical Products.

Les polymères d'origine naturelle, éventuellement modifiés, peuvent être choisis parmi la résine shellac, la gomme de sandaraque, les dammars, les élémis, les copals, les polymères cellulosiques, et leurs mélanges.

Selon un premier mode de réalisation de la composition selon l'invention, le polymère filmogène peut être présent sous la forme de particules en dispersion aqueuse, connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations NEOCRYL XK-90® , NEOCRYL A-1070® , NEOCRYL A-1090® , NEOCRYL BT-62® , NEOCRYL A-1079® , NEOCRYL A-523® par la société AVECIA-NEORESINS, DOW LATEX 432® par la société DOW CHEMICAL, DAITOSOL 5000 AD® par la société DAITO KASEY KOGYO; ou ben encore les dispersions aqueuses de polyuréthane vendues sous les dénominations NEOREZ R-981® , NEOREZ R-974® par la société AVECIA-NEORESINS, les AVALURE UR-405® , AVALURE UR-410® , AVALURE UR-425® , AVALURE UR-450® , SANCURE 875® , SANCURE 861® , SANCURE 878®, SANCURE 2060® par la société GOODRICH, IMPRANIL 85® par la société BAYER, AQUAMERE H-1511® par la société HYDROMER.

Comme dispersion aqueuse de polymère filmogène, on peut également utiliser les dispersions de polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthanes, les polyurées, les polyesters, les polyesteramides et/ou les alkydes. Ces polymères sont généralement appelés polymères hybrides.

Selon une deuxième variante de réalisation de la composition selon l'invention, le polymère filmogène peut être un polymère hydrosoluble et est donc présent dans la phase aqueuse de la composition sous forme solubilisée. Comme exemples de polymères filmogènes hydrosolubles, on peut citer

- les protéines comme les protéines d'origine végétale telles que les protéines de blé, de soja ; les protéines d'origine animale tels que les kératines, par exemples les hydrolysats de kératine et les kératines sulfoniques ;
- les polymères de chitine ou de chitosane anioniques, cationiques, amphotères ou non-ioniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères d'origine naturelle, éventuellement modifiés, tels que :

  . les gommes arabiques, la gomme de guar, les dérivés du xanthane, la gomme de karaya ;
  . les alginates et les carraghénanes ;
  . les glycoaminoglycanes, l'acide hyaluronique et ses dérivés ;
  . la résine shellac, la gomme de sandaraque, les dammars, les élémis, les copals;
  . l'acide désoxyribonucléique ;
  . les muccopolysaccharides tels que l'acide hyaluronique, les chondroïtines sulfate, et leurs mélanges.

Selon une autre variante de réalisation de la composition selon l'invention, le polymère filmogène peut être présent dans une phase grasse liquide comprenant des huiles ou solvants organiques tels que ceux décrits précédemment. Par "phase grasse liquide", on entend, au sens de l'invention, une phase grasse liquide à température ambiante (25°C) et pression atmosphérique (760 mm de Hg, soit $10^5$ Pa), composée d'un ou plusieurs corps gras liquides à température ambiante, appelés aussi huiles, généralement compatibles entre eux.

De préférence, la phase grasse liquide comprend une huile volatile, éventuellement en mélange avec une huile non volatile, les huiles pouvant être choisies parmi les huiles citées précédemment.

Selon un troisième mode de réalisation de la composition selon l'invention, le polymère filmogène peut être présent sous forme de particules, stabilisées en surface, dispersées dans la phase grasse liquide.

La dispersion de particules de polymère stabilisées en surface peut être fabriquée comme décrit dans le document EP-A-749747.

Les particules de polymère sont stabilisées en surface grâce à un stabilisant qui peut être un polymère séquence, un polymère greffé, et/ou un polymère statistique, seul ou en mélange.

Des dispersions de polymère filmogène dans la phase grasse liquide, en présence d'agent stabilisants, sont notamment décrites dans les documents EP-A-749746, EP-A-923928, EP-A-930060 dont le contenu est incorporé à titre de référence dans la présente demande.

La taille des particules de polymères en dispersion soit dans la phase aqueuse, soit dans la phase grasse liquide, peut aller de 5 nm à 600 nm, et de préférence de 20 nm à 300 nm.

Selon un quatrième mode de réalisation de la composition selon l'invention, le polymère filmogène peut être solubilisé dans la phase grasse liquide, on dit alors que le polymère filmogène est un polymère liposoluble.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester ) et d'au moins un autre monomère

qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui ont pour but qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2% de divinyl benzène, acétate de vinyle/octadécyl vinyl éther, réticulé avec 0,2% de tétraallyloxyéthane, acétate de vinyle/stéarate d'allyle, réticulé avec 0,2% de divinyl benzène, acétate de vinyle/octadécène-1 réticulé avec 0,2% de divinyl benzène et propionate d'allyle/stéarate d'allyle réticulé avec 0,2% de divinyl benzène.

Comme polymères filmogènes liposolubles, on peut également citer les copolymères liposolubles, et en particulier ceux résultant de la copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, polystéarate de vinyle réticulé à l'aide de divinylbenzène, de diallyléther ou de phtalate de diallyle, poly(méth)acrylate de stéaryle, polylaurate de vinyle, poly(méth)acrylate de lauryle, ces poly(méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en $C_2$-$C_{20}$, comme le polybutène, les alkylcelluloses avec un radical alkyle linéaire ou ramifié, saturé ou non en $C_1$ à $C_8$ comme l'éthylcellulose et la propylcellulose, les copolymères de la vinylpyrolidone (VP) et notamment les copolymères de la vinylpyrrolidone et d'alcène en $C_2$ à $C_{40}$ et mieux en $C_3$ à $C_{20}$. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

La composition selon l'invention peut comprendre un agent auxiliaire de filmification favorisant la formation d'un film avec le polymère filmogène. Un tel agent de filmification peut être choisi parmi tous les composés connus de l'homme du métier comme étant susceptibles de remplir la fonction recherchée, et notamment être choisi parmi les agents plastifiants et les agents de coalescence.

La composition selon l'invention peut également comprendre une matière colorante comme les matières colorantes pulvérulentes, les colorants liposolubles, les colorants hydrosolubles. Cette matière colorante peut être présente en une teneur allant de 0,01 % à 30 % en poids, par rapport au poids total de la composition.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C orange 5, le jaune quinoléine, le rocou. Les colorants hydrosolubles sont par exemple le jus de betterave, le bleu de méthylène.

La composition selon l'invention peut comprendre en outre des charges. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon®) (Orgasol® de chez Atochem) , de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.
Les charges peuvent être présentes à raison de 0,01 à 30% en poids, et de préférence 0,5% à 15% en poids.
La composition de l'invention peut comprendre, en outre, tout additif usuellement utilisé en cosmétique tels que les antioxydants, les charges, les conservateurs, les parfums, les neutralisants, les épaississants, les tensioactifs, les actifs cosmétiques ou dermatologiques comme par exemple des émollients, des hydratants, des vitamines, des filtres solaires, des agents plastifiants, des agents de coalescence et leurs mélanges. Ces additifs peuvent être présents dans la composition en une teneur allant de 0,01 à 20% du poids total de la composition et mieux allant de 0,01 à 10%.
Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.
La composition selon l'invention peut être fabriquée par les procédés connus, généralement utilisés dans le domaine cosmétique ou dermatologique.
L'invention a trait également a un procédé cosmétique de traitement ou de maquillage des matières kératiniques, comprenant l'application sur lesdites matières kératiniques de la composition, telle qu'elle est décrite plus haut.
L'invention concerne aussi un procédé de revêtement des cils comprenant l'application sur les cils de la composition décrite ci-dessus.
L'invention est également relative à l'utilisation de la composition, telle que décrite plus haut, pour obtenir un allongement des cils se situant exactement dans le prolongement de ceux-ci, ou pour conférer aux cils un effet d'allongement régulier.
L'invention a trait, en outre, à l'utilisation dans un mascara, de fibres, telles que définies plus haut, pour obtenir un allongement des cils se situant exactement dans le prolongement de ceux-ci ou pour conférer aux cils un effet d'allongement régulier.
Enfin, l'invention est relative à l'utilisation dans un mascara, de fibres, telles que définies plus haut, pour mimer, imiter, les cils naturels, ainsi qu'à l'utilisation d'une composition telle que décrite plus haut, telle qu'un mascara pour mimer, imiter, les cils naturels.
L'invention est illustrée plus en détails dans les exemples suivants :

**Exemple 1 :**

On a préparé un mascara ayant la composition suivante :

- Fibres de polyimide-amide de 2 mm de longueur vendue sous la dénomination KERMEL TECH par la société Rhodia 1 g
- Saponite (Veegum DGT de la société Vanderbilt) 10 g
- Oxyde de fer noir 7 g
- Propylène glycol 7 g
- Conservateurs qs
- Eau qsp 100 g

Le mascara s'applique facilement sur les cils et confère à ces derniers un effet d'allongement régulier : les fibres rigides sont fixées dans le prolongement des cils.

**Exemple 2 :**

On a préparé un mascara ayant la composition suivante :

- Fibres de polyamide de 3 mm de longueur coupée à partir de la fibre vendue sous la dénomination FIBRE NOMEX BRAND 430 par la par la société Dupont de Nemours 1 g

- Saponite (Veegum DGT de la société Vanderbilt) 10 g
- Oxyde de fer noir 7 g
- Propylène glycol 7 g
- Conservateurs qs
- Eau qsp 100 g

Le mascara s'applique facilement sur les cils et confère à ces derniers un effet d'allongement régulier : les fibres rigides sont fixées dans le prolongement des cils.

## Revendications

1. Composition cosmétique comprenant des fibres rigides, sensiblement rectilignes, d'un polymère synthétique dans un milieu physiologiquement acceptable, dans laquelle le polymère est choisi parmi les polyuréthanes, les polyesters, les polymères acryliques, les polyoléfines, les polyamides non aromatiques et les polyimides-amides aromatiques ; lesdites fibres étant telles que lorsque l'on observe au microscope, avec un objectif permettant un grossissement de 2,5 et avec une vision plein champ, un milieu dans lequel sont dispersées les fibres à une concentration des fibres de 1% en poids, au moins 50 % en nombre, de préférence au moins 75% en nombre, et mieux au moins 90% en nombre des fibres sont telles que l'angle formé entre la tangente à l'axe central longitudinal de la fibre à l'une des extrémités de la fibre et la droite reliant ladite extrémité au point sur l'axe central longitudinal de la fibre correspondant à la moitié de la longueur de fibre soit inférieur ou égal à 15°, et l'angle formé entre la tangente à l'axe central longitudinal de la fibre en un point situé à mi-longueur de la fibre et la droite reliant une des extrémités au point sur l'axe central longitudinal de la fibre correspondant à la moitié de la longueur de fibre soit inférieur ou égal à 15°, pour une même longueur de fibre allant de 0,8 mm à 5 mm, de préférence allant de 1 à 4 mm, de préférence encore de 1 à 3 mm, et mieux de 2 mm.

2. Composition selon la revendication 1, dans laquelle lesdits angles sont inférieurs ou égaux à 10°, de préférence inférieurs ou égaux à 5°.

3. Composition selon l'une quelconque des revendications précédentes, dans laquelle les fibres ont une longueur de 0,8 à 5 mm, de préférence de 1 à 4 mm et mieux de 1 à 3 mm.

4. Composition selon l'une quelconque des revendications précédentes, dans laquelle la section des fibres est comprise dans un cercle de diamètre (D) allant de 2 nm à 500 µm, de préférence de 100 nm à 100 µm, et mieux de 1 µm à 50 µm.

5. Composition selon l'une quelconque des revendications précédentes, dans laquelle les fibres ont une longueur L et un diamètre D du cercle dans lequel s'inscrit la section de la fibre, tel que le rapport L/D, soit dans la plage de 3,5 à 2 500, de préférence de 5 à 500, et mieux de 5 à 150.

6. Composition selon l'une quelconque des revendications précédentes, dans laquelle les fibres ont un titre dans la plage de 0,15 à 30 deniers, et, de préférence, de 0,18 à 18 deniers.

7. Composition selon l'une quelconque des revendications précédentes, dans laquelle les fibres sont présentes à une teneur de 0,01 % à 10 % en poids, par rapport au poids total de la composition, de préférence de 0,1 % à 5 % en poids, et mieux de 0,3 % à 3 % en poids.

8. Composition selon la revendication 1, dans laquelle les fibres sont des fibres de polyimide-amide aromatique, ledit polyimide-amide aromatique comprenant un motif récurrent de formule (I) :

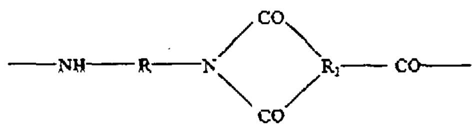

(I)

éventuellement, en outre, un motif récurrent de formule (II) :

-NH-R-NH-CO-$R_2$-CO- (II)

éventuellement, en outre, un motif récurrent de formule (III) :

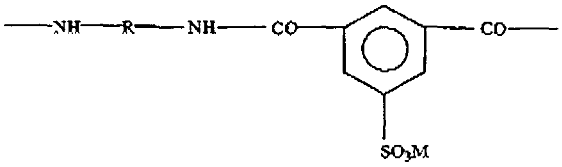

(III)

éventuellement, en outre, un motif récurrent de formule (IV) :

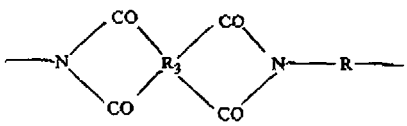

**9.** Composition selon la revendication 8, dans laquelle $R_1$ représente :

un groupe

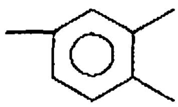

,

un groupe

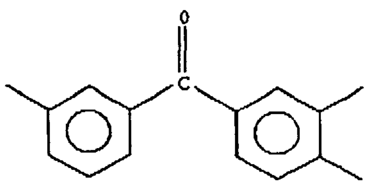

,

ou un groupe

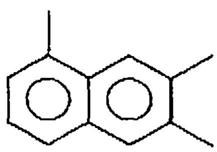

.

**10.** Composition selon l'une quelconque des revendications 8 et 9, dans laquelle R est choisi parmi les groupes :

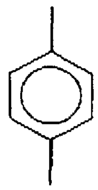 ; 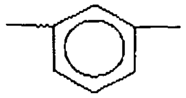 ; 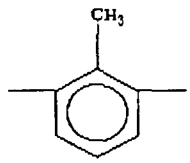 ; 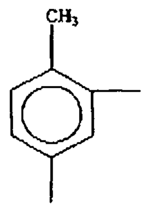 ;

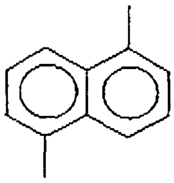 ; 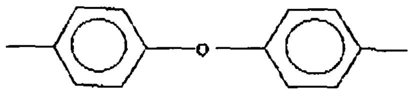 ; 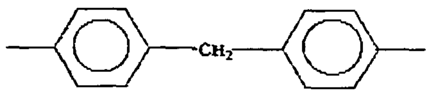 ;

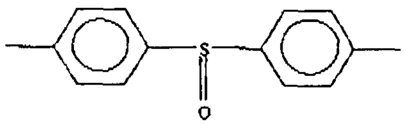 ; 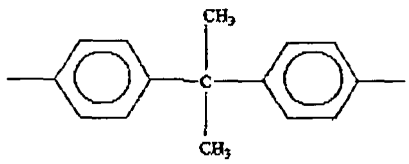 ;

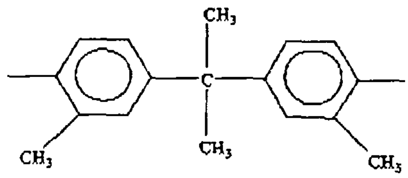 .

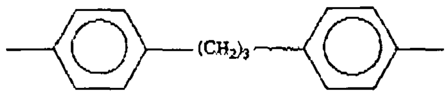 , 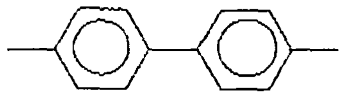

**11.** Composition selon l'une quelconque des revendications 8 à 10, dans laquelle $R_2$ est un groupe de formule :

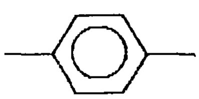 ; 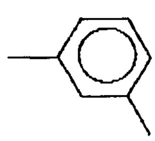 ; 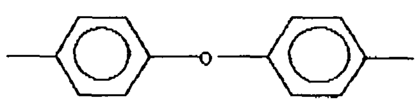

**12.** Composition selon l'une quelconque des revendications 8 à 11, dans laquelle $R_3$ est choisi parmi les groupes de formule :

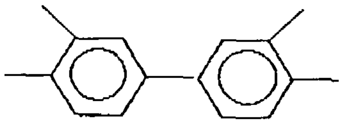 ; 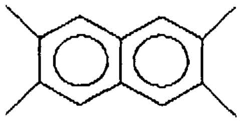 - 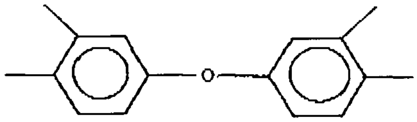 ;

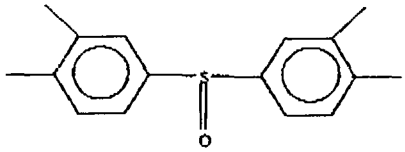 ; 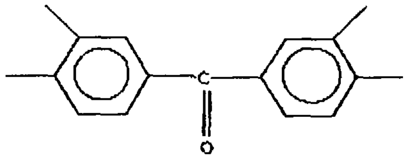 ;

**13.** Composition selon l'une quelconque des revendications 8 à 12, dans laquelle le polyimide-amide est obtenu par polymérisation du tolyulène diisocyanate et de l'anhydride triméllitique et comprend des motifs répétitifs de formule :

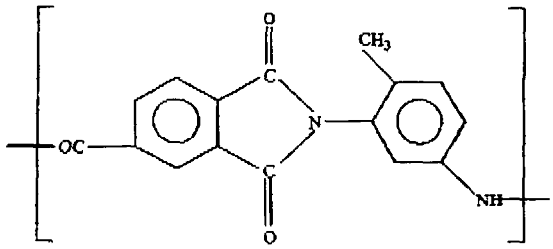

**14.** Composition selon l'une quelconque des revendications 1 à 13, dans laquelle les fibres ont traitées en surface et/ou enrobées, par exemple, par un agent actif et/ou un colorant et/ou un pigment.

**15.** Composition selon l'une quelconque des revendications 1 à 13, dans laquelle un agent actif et/ou pigment et/ou colorant est incorporé dans la masse du polymère formant les fibres.

**16.** Composition selon l'une quelconque des revendications précédentes, dans laquelle le milieu physiologiquement acceptable est un milieu cosmétique hydrophile ou lipophile.

**17.** Composition selon l'une quelconque des revendications précédentes, comprenant de l'eau ou un mélange d'eau et de solvant(s) organique(s) hydrophile(s).

**18.** Composition selon la revendication 17, dans laquelle le(s) solvant(s) organique(s) hydrophile(s) est (sont) choisi(s) parmi les monoalcools ayant de 2 à 5 atomes de carbone, les polyols ayant de 2 à 8 atomes de carbone, les cétones en $C_3$-$C_4$ et les aldéhydes en $C_2$-$C_4$.

**19.** Composition selon l'une quelconque des revendications 17 et 18, dans laquelle l'eau ou le mélange d'eau et de solvant(s) organique (s) hydrophile(s) est présent en une teneur allant de 0,1% à 90% en poids, par rapport au poids total de la composition, et de préférence de 0,1% à 60% en poids.

**20.** Composition selon l'une quelconque des revendications précédentes qui comprend une phase grasse.

**21.** Composition selon la revendication 20 qui est une composition anhydre.

**22.** Composition selon la revendication 20 ou la revendication 21, dans laquelle la phase grasse comprend un corps gras choisis parmi les huiles, les solvants organiques, les cires, les corps gras pâteux, et leurs mélanges.

**23.** Composition selon l'une quelconque des revendications 20 à 22, dans laquelle la phase grasse comprend au moins une huile volatile.

**24.** Composition selon la revendication 23, dans laquelle l'huile volatile est choisie parmi les huiles hydrocarbonées ayant de 8 à 16 atomes de carbone.

**25.** Composition selon la revendication 24, dans laquelle l'huile volatile est présente en une teneur allant de 0,1% à 98% en poids, par rapport au poids total de la composition, de préférence de 1% à 65% en poids.

**26.** Composition selon l'une quelconque des revendications précédentes, **caractérisée en ce qu'**elle comprend un polymère filmogène.

**27.** Composition selon la revendication 26, dans laquelle le polymère filmogène est choisi dans le groupe formé par les polymères vinyliques, les polyuréthanes, les polyesters, les polyamides, les polyurées, et les polymères cellulosiques.

**28.** Composition selon la revendication 27, dans laquelle le polymère filmogène est présent en une teneur en matières sèches de polymère allant de 0,1% à 60% en poids par rapport au poids total de la composition, de préférence de 0,5% à 40% en poids, et mieux de 1% à 30% en poids.

**29.** Composition selon l'une quelconque des revendications précédentes, **caractérisée en ce qu'**elle comprend une matière colorante

**30.** Composition selon la revendication 29, dans laquelle la matière colorante est choisie parmi les pigments, les nacres, les colorants liposolubles, et les colorants hydrosolubles.

**31.** Composition selon la revendication 29 ou la revendication 30, dans laquelle la matière colorante est présente en une teneur allant de 0,01% à 30% en poids, par rapport au poids total de la composition.

**32.** Composition selon l'une quelconque des revendications précédentes, **caractérisée en ce qu'**elle comprend un additif cosmétique choisi parmi les antioxydants, les charges, les conservateurs, les parfums, les neutralisants, les épaississants, les tensioactifs, les actifs cosmétiques ou dermatologiques, les agents plastifiants, les agents de coalescence et leurs mélanges.

**33.** Composition selon l'une quelconque des revendications précédentes, qui est une composition de maquillage, une base de maquillage, une composition dite « top-coat » à appliquer sur un maquillage, ou une composition de traitement, de soin, des matières, fibres, kératinique.

**34.** Composition selon l'une quelconque des revendications précédentes qui est une composition de revêtement des cils, notamment une composition de maquillage des cils, encore appelé mascara, une composition à appliquer sur un maquillage des cils, dite encore top-coat, ou bien encore une composition de traitement des cils, notamment des cils d'êtres humains ou des faux cils.

**35.** Composition selon la revendication 34, qui est un mascara.

**36.** Procédé cosmétique de traitement ou de maquillage des matières kératiniques comprenant l'application sur lesdites matières kératiniques d'une composition selon l'une quelconque des revendications 1 à 35.

**37.** Procédé de revêtement des cils comprenant l'application sur les cils d'une composition selon l'une quelconque des revendications 1 à 35.

**38.** Utilisation d'une composition selon l'une quelconque des revendications 1 à 35, pour obtenir un allongement des cils se situant exactement dans le prolongement de ceux-ci ou pour conférer aux cils un effet d'allongement régulier.

**39.** Utilisation dans un mascara, de fibres, telles que définies dans l'une quelconque des revendications 1 à 35 pour obtenir un allongement des cils se situant exactement dans le prolongement de ceux-ci ou pour conférer aux cils un effet d'allongement régulier.

**40.** Utilisation dans un mascara, de fibres, telles que définies dans l'une quelconque des revendications 1 à 35, pour mimer, imiter les cils naturels.

**41.** Utilisation d'une composition selon l'une quelconque des revendications 1 à 35, pour mimer, imiter, les cils naturels.

**Claims**

**1.** Cosmetic composition comprising rigid, substantially rectilinear fibres, of a synthetic polymer in a physiologically acceptable medium, wherein the polymer is selected from polyurethanes, polyesters, acrylic polymers, polyolefins, non-aromatic polyamides and aromatic polyimides-amides; said fibres being such that, when observing with a microscope, with a lens enabling a magnification of 2.5 and with full field vision, a medium wherein the fibres are dispersed at a fibre concentration of 1% by weight, at least 50% in number, preferentially at least 75% in number, and more preferentially at least 90% in number of the fibres are such that the angle formed between the tangent to the central longitudinal axis of the fibre at one of the ends of the fibre and the line joining said end to the point on the central longitudinal axis of the fibre corresponding to half the fibre length is less than or equal to 15°, and the angle formed between the tangent to the central longitudinal axis of the fibre at a point located at mid-length of the fibre and the line joining one of the ends to the point on the central longitudinal axis of the fibre corresponding to half the fibre length is less than or equal to 15°, for the same fibre length ranging from 0.8 mm to 5 mm, preferably ranging from 1 to 4 mm, more preferably from 1 to 3 mm, and even more preferably 2 mm.

**2.** Composition according to claim 1, wherein said angles are less than or equal to 10°, preferably less than or equal to 5°.

**3.** Composition according to any one of the preceding claims, wherein the fibres have a length of 0.8 to 5 mm, preferably from 1 to 4 mm and more preferably from 1 to 3 mm.

**4.** Composition according to any one of the preceding claims, wherein the cross-section of the fibres is within a circle of diameter (D) ranging from 2 nm to 500 μm, preferably from 100 nm to 100 μm, and more preferably from 1 μm to 50 μm.

**5.** Composition according to any one of the preceding claims, wherein the fibres have a length L and a diameter D of the circle wherein the cross-section of the fibre is inscribed, such that the ratio L/D, is in the range from 3.5 to 2500, preferably from 5 to 500, and more preferably from 5 to 150.

**6.** Composition according to any one of the preceding claims, wherein the fibres have a yarn count in the range from 0.15 to 30 deniers, and, preferably, from 0.18 to 18 deniers.

**7.** Composition according to any one of the preceding claims, wherein the fibres are present at a content of 0.01% to 10% by weight, with respect to the total weight of the composition, preferably from 0.1% to 5% by weight, and more preferably from 0.3% to 3% by weight.

**8.** Composition according to claim 1, wherein the fibres are aromatic polyimide-amide fibres, said aromatic polyimide-amide comprising a repeating structural unit having the formula (I):

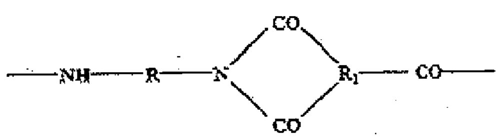

(I)

optionally, in addition, a repeating structural unit having the formula (II);

$$-NH-R-NH-CO-R_2-CO- \quad (II)$$

optionally, in addition, a repeating structural unit having the formula (III):

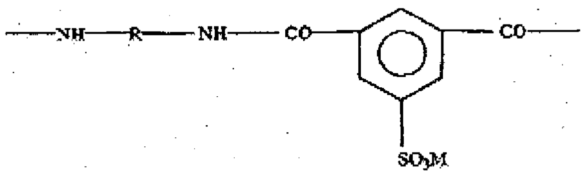 (III)

optionally, in addition, a reapeating structural unit having the formula (IV):

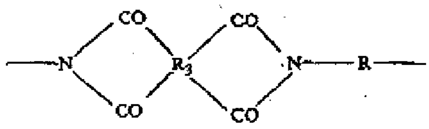

**9.** Composition according to claim 8, wherein $R_1$ represents:

a

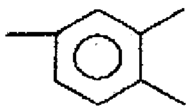

group , a

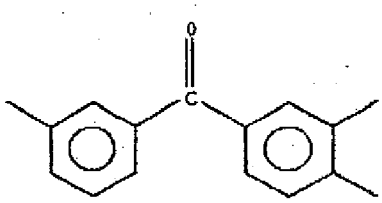

group , or a

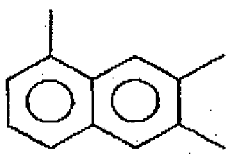

group.

**10.** Composition according to any one of claims 8 and 9, wherein R is selected from the following groups:

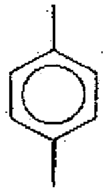 ; 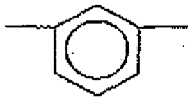 ; 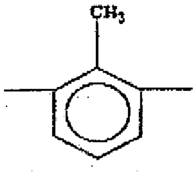 ; 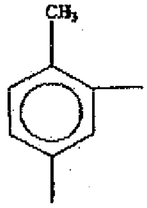 ;

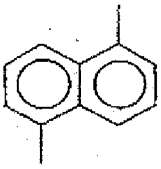 ; 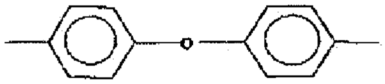 ; 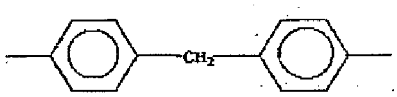 ;

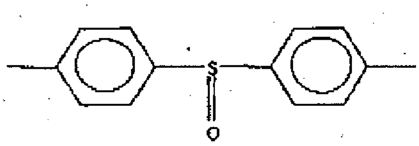 ; 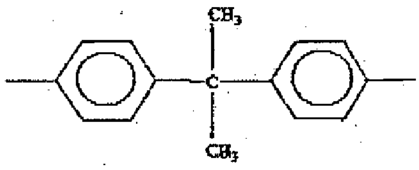 ;

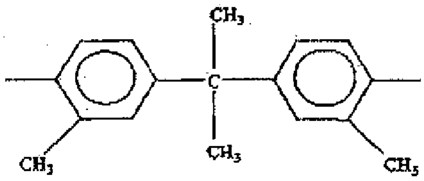 .

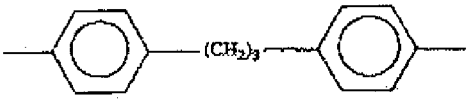 , 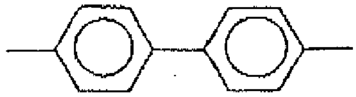

**11.** Composition according to any one of claims 8 to 10, wherein $R_2$ is a group having the formula:

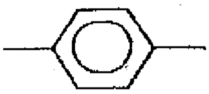 ; 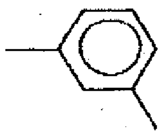 ; 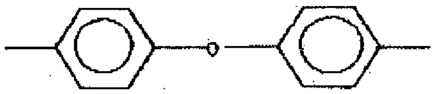

**12.** Composition according to any one of claims 8 to 11, wherein $R_3$ is selected from the groups having the formula:

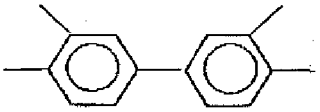 ; 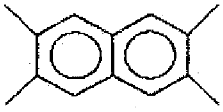 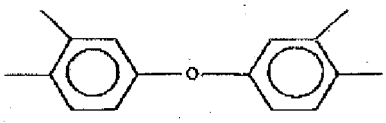 ;

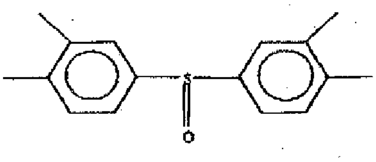 ; 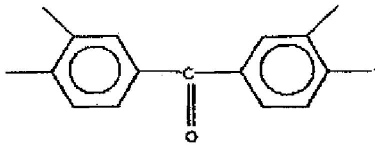 ;

13. Composition according to any one of claims 8 to 12, wherein the polyimide-amide is obtained by polymerization of toluene diisocyanate and trimellitic anhydride and comprises repeating structural units having the formula:

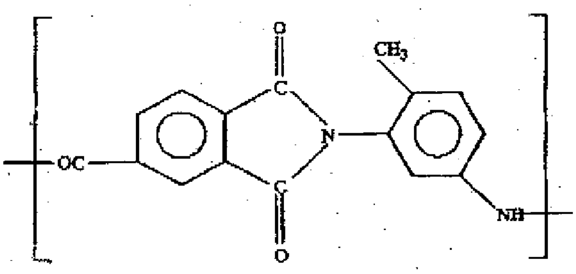

14. Composition according to any one of claims 1 to 13, wherein the fibres are treated on the surface and/or coated, for example, by an active agent and/or a dye and/or a pigment.

15. Composition according to any one of claims 1 to 13, wherein an active agent and/or pigment and/or dye is incorporated into the bulk of the polymer forming the fibres.

16. Composition according to any one of the preceding claims, wherein the physiologically acceptable medium is a hydrophilic or lipophilic cosmetic medium.

17. Composition according to any one of the preceding claims, comprising water or a mixture of water and hydrophilic organic solvent(s).

18. Composition according to claim 17, wherein the hydrophilic organic solvent(s) is/are selected from monoalcohols having 2 to 5 carbon atoms, polyols having 2 to 8 carbon atoms, $C_3$-$C_4$ ketones and $C_2$-$C_4$ aldehydes.

19. Composition according to any one of claims 17 and 18, wherein the water or mixture of water and hydrophilic organic solvent(s) is present at a content ranging from 0.1% to 90% by weight, with respect to the total weight of the composition, and preferably from 0.1% to 60% by weight.

20. Composition according to any one of the preceding claims which comprises a fatty phase.

21. Composition according to claim 20 which is an anhydrous composition.

22. Composition according to claim 20 or claim 21, wherein the fatty phase comprises a fatty substance selected from oils, organic solvents, waxes, pasty fatty substances, and mixtures thereof.

23. Composition according to any one of claims 20 to 22, wherein the fatty phase comprises at least one volatile oil.

**24.** Composition according to claim 23, wherein the volatile oil is selected from hydrocarbon oils having 8 to 16 carbon atoms.

**25.** Composition according to claim 24, wherein the volatile oil is present at a content ranging from 0.1% to 98% by weight, with respect to the total weight of the composition, preferably from 1% to 65% by weight.

**26.** Composition according to any one of the preceding claims, **characterised in that** it comprises a film-forming polymer.

**27.** Composition according to claim 26, wherein the film-forming polymer is selected in the group formed by vinyl polymers, polyurethanes, polyesters, polyamides, polyureas, and cellulose polymers.

**28.** Composition according to claim 27, wherein the film-forming polymer is present in a dry polymer matter content ranging from 0.1% to 60% by weight with respect to the total weight of the composition, preferably from 0.5% to 40% by weight, and more preferably from 1% to 30% by weight.

**29.** Composition according to any one of the preceding claims, **characterised in that** it comprises a dye stuff.

**30.** Composition according to claim 29, wherein the dye substance is selected from pigments, nacres, liposoluble dyes, and water-soluble dyes.

**31.** Composition according to claim 29 or claim 30, wherein the dye stuff is present in a content ranging from 0.01% to 30% by weight, with respect to the total weight of the composition.

**32.** Composition according to any one of the preceding claims, **characterised in that** it comprises a cosmetic additive selected from antioxidants, fillers, preservatives, fragrances, neutralizing agents, thickeners, surfactants, cosmetic or dermatological active substances, plasticizers, coalescence agents and mixtures thereof.

**33.** Composition according to any one of the preceding claims, which is a make-up composition, a make-up foundation, a so-called "top-coat" composition to be applied over a make-up, or keratinous materials, fibre, treatment, care composition.

**34.** Composition according to any one of the preceding claims which is an eyelashes coating composition, particularly an eyelash makeup composition, also referred to as mascara, a composition to be applied over an eyelash makeup, also referred to as a top-coat, or an eyelash treatment composition, particularly for human eyelashes or false eyelashes.

**35.** Composition according to claim 34, which is a mascara.

**36.** Cosmetic treatment or makeup method of keratinous materials comprising the application to said keratinous materials of a composition according to any one of claims 1 to 35.

**37.** Eyelash coating method comprising the application on the eyelashes of a composition according to any one of claims 1 to 35.

**38.** Use of a composition according to any one of claims 1 to 35, to obtain a lengthening of the eyelashes that is exactly in line with them or to give the eyelashes an even lengthening effect.

**39.** Use in a mascara of fibres as defined in any one of claims 1 to 35 to obtain a lengthening of the eyelashes that is exactly in line with them or to give the eyelashes an even lengthening effect.

**40.** Use in a mascara of fibres, as defined in any one of claims 1 to 35, to mimic, imitate natural eyelashes.

**41.** Use of a composition according to any one of claims 1 to 35, to mimic, imitate natural eyelashes.

**Patentansprüche**

**1.** Kosmetische Zusammensetzung, die in einem physiologisch akzeptablen Medium in etwa geradlinige, starre Fasern

eines synthetischen Polymers enthält, wobei das Polymer unter den Polyurethanen, Polyestern, Acrylpolymeren, Polyolefinen, Polyamiden, die nicht aromatisch sind, und aromatischen Polyimiden-amiden ausgewählt ist; und wobei die Fasern so vorliegen, dass, wenn ein Medium, in dem die Fasern in einer Konzentration der Fasern von 1 Gew.-% dispergiert sind, unter dem Mikroskop mit einem Objektiv, das eine 2,5-fache Vergrößerung zulässt, unter vollem Sehfeld betrachtet wird, mindestens 50 % der Anzahl der Fasern, vorzugsweise mindestens 75 % der Anzahl der Fasern und besser mindestens 90 % der Anzahl der Fasern so sind, dass für eine Faserlänge, die im Bereich von 0,8 bis 5 mm, vorzugsweise 1 bis 4 mm und noch bevorzugter 1 bis 3 mm liegt und noch besser 2 mm beträgt, der Winkel zwischen der Tangente an die zentrale Faserlängsachse an einem Ende der Faser und der dieses Faserende mit dem bei halber Faserlänge auf der zentralen Faserlängsachse gelegenen Punkt verbindenden Gerade kleiner oder gleich 15° ist und der Winkel zwischen der Tangente an die zentrale Faserlängsachse bei einem darauf auf halber Faserlänge gelegenen Punkt und der eines der Enden mit dem bei halber Faserlänge auf der zentralen Faserlängsachse gelegenen Punkt verbindenden Gerade kleiner oder gleich 15° ist.

2. Zusammensetzung nach Anspruch 1, bei der die genannten Winkel kleiner oder gleich 10° und vorzugsweise kleiner oder gleich 5° sind.

3. Zusammensetzung nach einem der vorhergehenden Ansprüche, bei der die Fasern eine Länge von 0,8 bis 5 mm, vorzugsweise 1 bis 4 mm und besser 1 bis 3 mm besitzen.

4. Zusammensetzung nach einem der vorhergehenden Ansprüche, bei der der Querschnitt der Fasern in einen Kreis mit einem Durchmesser (D) von 2 nm bis 500 μm, vorzugsweise 100 nm bis 100 μm und besser 1 bis 50 μm einbeschrieben werden kann.

5. Zusammensetzung nach einem der vorhergehenden Ansprüche, bei der die Fasern eine solche Länge L besitzen und der Durchmesser D des Kreises, in den der Querschnitt der Faser einbeschrieben werden kann, so ist, dass das Verhältnis L/D im Bereich von 3,5 bis 2 500, vorzugsweise 5 bis 500 und besser 5 bis 150 liegt.

6. Zusammensetzung nach einem der vorhergehenden Ansprüche, bei der die Fasern einen Titer im Bereich von 0,15 bis 30 Denier und vorzugsweise 0,18 bis 18 Denier aufweisen.

7. Zusammensetzung nach einem der vorhergehenden Ansprüche, bei der die Fasern in einer Menge von 0,01 bis 10 Gew.-%, bezogen auf das Gesamtgewicht der Zusammensetzung, vorzugsweise 0,1 bis 5 Gew.-% und besser 0,3 bis 3 Gew.-% enthalten sind.

8. Zusammensetzung nach Anspruch 1, bei der die Fasern aromatische Polyimid-amid-Fasern sind, wobei das aromatische Polyimid-amid eine Wiederholungseinheit der folgenden Formel (I):

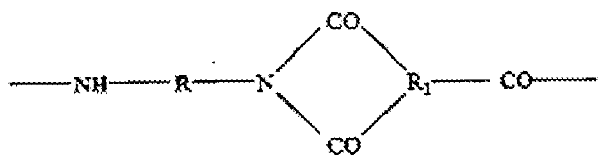

(I)

gegebenenfalls ferner eine Wiederholungseinheit der folgenden Formel (II):

$$-NH-R-NH-CO-R_2-CO- \quad (II)$$

gegebenenfalls ferner eine Wiederholungseinheit der folgenden Formel (III):

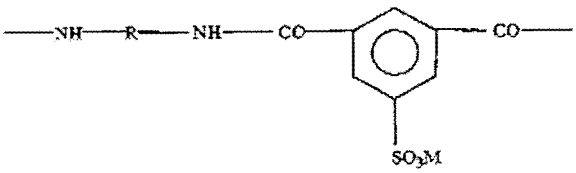

(III)

und gegebenenfalls ferner eine Wiederholungseinheit der folgenden Formel (IV) aufweist:

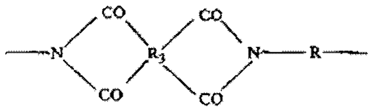

**9.** Zusammensetzung nach Anspruch 9, wobei $R_1$ bedeutet:

eine Gruppe

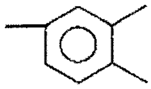

,

eine Gruppe

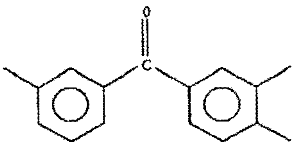

,

oder eine Gruppe

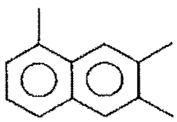

.

**10.** Zusammensetzung nach einem der Ansprüche 8 und 9, wobei R unter den folgenden Gruppen ausgewählt ist:

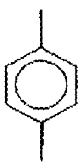

;

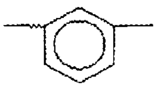

;

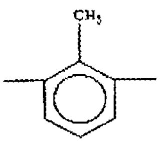

;

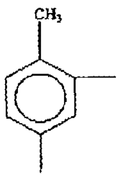

;

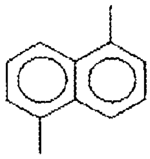 ; 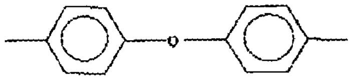 ; 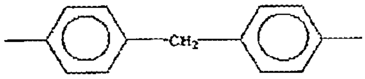 ;

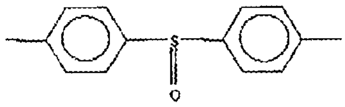 ; 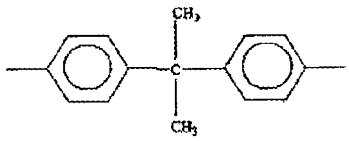 ;

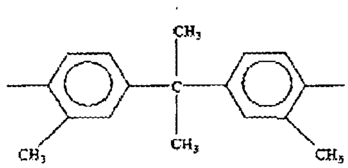 .

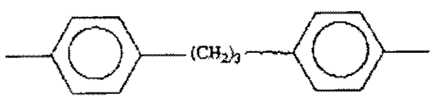 , 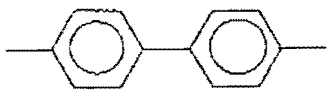

**11.** Zusammensetzung nach einem der Ansprüche 8 bis 10, wobei $R_2$ eine Gruppe der folgenden Formel ist:

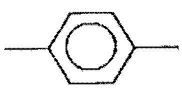 ; 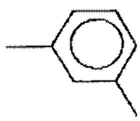 ; 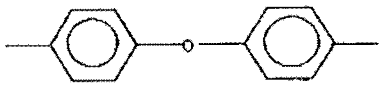

**12.** Zusammensetzung nach einem der Ansprüche 8 bis 11, wobei $R_3$ unter den Gruppen der folgenden Formel ausgewählt ist:

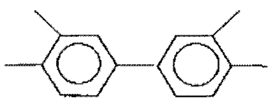 ; 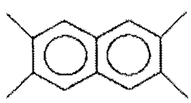 . 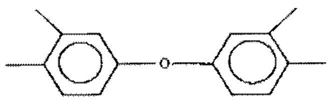 ;

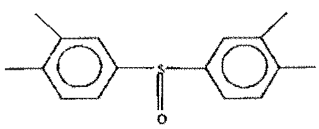 ; 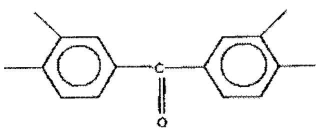 ;

**13.** Zusammensetzung nach einem der Ansprüche 8 bis 12, wobei das Polyimid-amid durch Polymerisation von Toluylendiisocyanat und Trimellitsäureanhydrid erhalten wird und Wiederholungseinheiten der folgenden Formel aufweist:

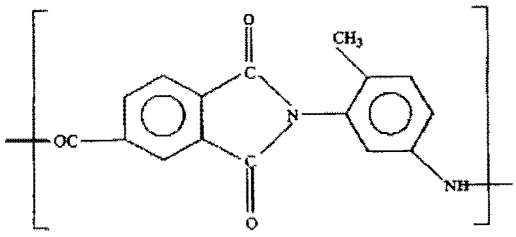

**14.** Zu- sammensetzung nach einem der Ansprüche 1 bis 13, wobei die Fasern an der Oberfläche behandelt und/oder umhüllt sind, beispielsweise mit einem Wirkstoff und/oder einem Farbstoff und/oder einem Pigment.

**15.** Zusammensetzung nach einem der Ansprüche 1 bis 13, wobei der Wirkstoff und/oder das Pigment und/oder der Farbstoff in die Masse des Polymers, das die Fasern bildet, eingebracht werden.

**16.** Zusammensetzung nach einem der vorhergehenden Ansprüche, wobei das physiologisch akzeptable Medium ein hydrophiles oder lipophiles kosmetisches Medium ist.

**17.** Zusammensetzung nach einem der vorhergehenden Ansprüche, die Wasser oder ein Gemisch von Wasser und einem oder mehreren hydrophilen organischen Lösemittel(n) enthält.

**18.** Zusammensetzung nach Anspruch 17, wobei das oder die hydrophile(n) organische(n) Lösemittel unter den Monoalkoholen mit 2 bis 5 Kohlenstoffatomen, Polyolen mit 2 bis 8 Kohlenstoffatomen, Ketonen mit 3 bis 4 Kohlenstoffatomen und Aldehyden mit 2 bis 4 Kohlenstoffatomen ausgewählt ist.

**19.** Zusammensetzung nach einem der Ansprüche 17 und 18, wobei das Wasser oder das Gemisch von Wasser und einem oder mehreren hydrophilen organischen Lösemittel(n) in einem Mengenanteil enthalten ist, der im Bereich von 0,1 bis 90 Gew.-%, bezogen auf das Gesamtgewicht der Zusammensetzung, und vorzugsweise 0,1 bis 60 Gew.-% liegt.

**20.** Zusammensetzung nach einem der vorhergehenden Ansprüche, die eine Fettphase enthält.

**21.** Zusammensetzung nach Anspruch 20, bei der es sich um eine wasserfreie Zusammensetzung handelt.

**22.** Zusammensetzung nach Anspruch 20 oder Anspruch 21, bei der die Fettphase eine Fettsubstanz enthält, die unter den Ölen, organischen Lösemitteln, Wachsen, pastösen Fettsubstanzen und deren Gemischen ausgewählt ist.

**23.** Zusammensetzung nach einem der Ansprüche 20 bis 22, wobei die Fettphase mindestens ein flüchtiges Öl enthält.

**24.** Zusammensetzung nach Anspruch 23, wobei das flüchtige Öl unter den Kohlenwasserstoffölen mit 8 bis 16 Kohlenstoffatomen ausgewählt ist.

**25.** Zusammensetzung nach Anspruch 24, wobei das flüchtige Öl in einem Mengenanteil von 0,1 bis 98 Gew.-%, bezogen auf das Gesamtgewicht der Zusammensetzung, und vorzugsweise 1 bis 65 Gew.-% enthalten ist.

**26.** Zusammensetzung nach einem der vorhergehenden Ansprüche, **dadurch gekennzeichnet, dass** sie ein filmbildendes Polymer enthält.

**27.** Zusammensetzung nach Anspruch 26, bei der das filmbildende Polymer unter den Vinylpolymeren, Polyurethanen, Polyestern, Polyamiden, Polyharnstoffen und Cellulosepolymeren ausgewählt ist.

**28.** Zusammensetzung nach Anspruch 27, wobei das filmbildende Polymer in einem Trockensubstanzgehalt des Polymers von 0,1 bis 60 Gew.-%, bezogen auf das Gesamtgewicht der Zusammensetzung, vorzugsweise 0,5 bis 40 Gew.-% und noch bevorzugter im Bereich von 1 bis 30 Gew.-% enthalten ist.

**29.** Zusammensetzung nach einem der vorhergehenden Ansprüche, **dadurch gekennzeichnet, dass** sie ein Farbmittel enthält.

**30.** Zusammensetzung nach Anspruch 29, wobei das Farbmittel unter den Pigmenten, Perlglanzstoffen, fettlöslichen Farbstoffen und wasserlöslichen Farbstoffen ausgewählt ist.

**31.** Zusammensetzung nach Anspruch 29 oder nach Anspruch 30, wobei das Farbmittel in einem Mengenanteil von 0,01 bis 30 Gew.-%, bezogen auf das Gesamtgewicht der Zusammensetzung, enthalten ist.

**32.** Zusammensetzung nach einem der vorhergehenden Ansprüche, **dadurch gekennzeichnet, dass** sie einen kosmetischen Zusatzstoff enthält, der unter den Antioxidantien, Füllstoffen, Konservierungsmitteln, Parfums, Neutralisationsmitteln, Verdickungsmitteln, grenzflächenaktive Stoffen, kosmetischen oder dermatologischen Wirkstoffen, Weichmachern, Koaleszenzmitteln und deren Gemischen ausgewählt ist.

**33.** Zusammensetzung nach einem der vorhergehenden Ansprüche, bei der es sich um eine Zusammensetzung zum Schminken, eine Make-up-Grundlage, einen so genannten "Top-coat", der auf eine Zusammensetzung zum Schminken aufgetragen wird, oder eine Zusammensetzung für die Behandlung, die Pflege von Keratinsubstanzen, Keratinfasern, handelt.

**34.** Zusammensetzung nach einem der vorhergehenden Ansprüche, bei der es sich um eine Zusammensetzung zum Überziehen von Wimpern, insbesondere eine Zusammensetzung zum Schminken von Wimpern, die auch als Mascara bezeichnet wird, eine Zusammensetzung, die auf die geschminkten Wimpern aufgetragen wird und auch als Top-coat bezeichnet wird, oder eine Zusammensetzung zur Behandlung der Wimpern, insbesondere von menschlichen Wimper oder falschen Wimpern, handelt.

**35.** Zusammensetzung nach Anspruch 34, die eine Mascara ist.

**36.** Kosmetisches Verfahren zur Behandlung oder zum Schminken von Keratinsubstanzen, das das Aufbringen einer Zusammensetzung nach einem der Ansprüche 1 bis 35 auf die Keratinsubstanzen umfasst.

**37.** Verfahren zum Überziehen von Wimpern, das das Aufbringen einer Zusammensetzung nach einem der Ansprüche 1 bis 35 auf die Wimpern umfasst.

**38.** Verwendung einer Zusammensetzung nach einem der Ansprüche 1 bis 35, um eine Verlängerung der Wimpern zu erreichen, die genau in ihrer Verlängerung besteht, oder um die Wimpern länger wirken zu lassen.

**39.** Verwendung von Fasern, wie sie in einem der Ansprüche 1 bis 35 definiert sind, in einer Mascara, um eine Verlängerung der Wimpern zu erreichen, die in ihrer Verlängerung besteht, oder um die Wimpern ebenmässig länger wirken zu lassen.

**40.** Verwendung von Fasern, wie sie in einem der Ansprüche 1 bis 35 definiert sind, in einer Mascara, um natürliche Wimpern vorzutäuschen, nachzuahmen.

**41.** Verwendung einer Zusammensetzung nach einem der Ansprüche 1 bis 35, um natürliche Wimpern vorzutäuschen, nachzuahmen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- JP 3153613 A
- JP 57158714 A
- JP 9263518 A
- JP 6009340 A
- JP 7179323 A
- FR 2817477 A
- EP 1201221 A
- EP 1053742 A
- EP 1208836 A
- EP 911217 A
- EP 686858 A
- US 5472798 A
- US 3802841 A
- FR 2079785 A
- EP 0360728 A1
- EP 0549494 A
- EP 847752 A
- EP 557196 A
- EP 1048282 A
- EP 749747 A
- EP 749746 A
- EP 923928 A
- EP 930060 A
- FR 2232303 A


### Littérature non-brevet citée dans la description


- Encyclopedia of Chemical Technology, KIRK-OTHMER. Encyclopedia of Chemical Technology, KIRK-OTHMER. WILEY, 1979, vol. 22, 333-432